# EUROPÄISCHE PATENTANMELDUNG

(11) **EP 0 754 685 A2**
(43) Veröffentlichungstag der Anmeldung: **22.01.1997**
(21) Anmeldenummer: 96111077.2
(22) Anmeldetag: 10.07.1996
(51) Int. Cl.: C07D 239/34, C09K 19/34, C07D 239/26, C07D 213/63, C07D 213/24

(54) **Derivate von alkoxyalkoxysubstituierten Stickstoff-Heterocyclen**

(30) Priorität: 17.07.1995 CH 2095/95
(71) Anmelder: Rolic AG, 4058 Basel (CH)
(72) Erfinder: Kelly, Stephen, Beverley HU17 8XA, East Yorkshire (GB)
(74) Vertreter: Kjellsaa-Berger, Hanny, Dr.

(57) **Zusammenfassung**

In der vorliegenden Erfindung werden Verbindungen der allgemeinen Formel 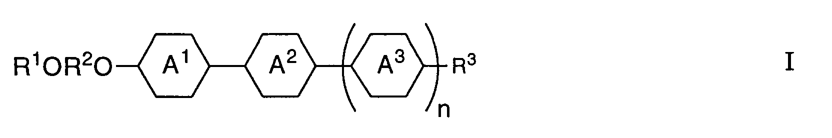 worin
- R¹: geradkettiges, unsubstituiertes oder ein- oder mehrfach mit Halogen substituiertes Alkyl oder Alkenyl mit 1 bis 8 Kohlenstoffatomen;
- R²: geradkettiges, unsubstituiertes oder ein- oder mehrfach mit Halogen substituiertes Alkylen mit 2 bis 8 Kohlenstoffatomen;
- R³: geradkettiges, unsubstituiertes oder ein- oder mehrfach mit Halogen substituiertes Alkyl, Alkoxy, Alkanoyloxy, Alkenyl, Alkenyloxy oder Alkenoyloxy mit 4 bis 12 Kohlenstoffatomen, in welchen eine oder mehrere nicht benachbarte Methylengruppen durch -O- ersetzt sein können;
- A¹, A² und A³: unabhängig voneinander unsubstituiertes oder ein- oder mehrfach mit Halogen substituiertes 1,4-Phenylen, oder Pyridin-2,5-diyl oder Pyrimidin-2,5-diyl, und A³ auch trans-1,4-Cyclohexylen bedeuten;
und
- n: 0 oder 1 ist;
mit der Massgabe, dass mindestens einer der Ringe A¹, A² oder A³ Pyridin-2,5-diyl oder Pyrimidin-2,5-diyl bedeutet; und falls n = 0 und A² Pyrimidin-2,5-diyl ist, R³ Alkenyl, Alkenyloxy, Alkanoyloxy oder Alkenoyloxy darstellt;
deren Herstellung und Verwendung in elektro-optischen Vorrichtungen beschrieben, sowie Mischungen, die solche Verbindungen enthalten.

## Beschreibung

Die vorliegende Erfindung betrifft Derivate von Alkoxyalkoxysubstituierten Stickstoff-Heterocyclen, flüssigkristalline Gemische, die solche Verbindungen enthalten, sowie deren Verwendung für elektrooptische Zwecke.

Flüssige Eristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gas/Wirt-Zellen mit verdrillt nematischer ("twisted nematic") Struktur, STN-Zellen ("super twisted nematic"), SBE-Zellen ("super birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Elektro-optische Vorrichtungen, die nematische Flüssigkristalle benützen, wie zum Beispiel TN-, STN- und TN-TFT-Zellen ("twisted nematic thin film transistor), weisen eine Reihe von Nachteilen auf, wie zum Beispiel lange Schaltzeiten oder eine starke Blickwinkelabhängikeit des Kontrastes, was bewirkt, dass sie sich für komplizierte, hoch informative Anzeige-Vorrichtungen, wie zum Beispiel videokompatible Projektionssysteme für Fernsehbildschirme nicht eignen. Ferroelektrische elektrooptische Vorrichtungen, wie zum Beispiel SSF- (surface stabilized ferroelectric) oder SBF-Zellen (surface bistabilized ferroelectric), können zwar sehr schnelle Schaltzeiten aufweisen, es ist aber bisher nicht gelungen, die für einfache Erzeugung von Farben erforderlichen Graustufen zu erzeugen; ein weiterer Nachteil der bisherigen ferroelektrischen Vorrichtungen sind die sogenannten 'Ghost Effects', die wegen statischer elektrischer Ladung auftreten und zu Verzerrungen der gezeigten Information führen kann. Im Gegensatz dazu zeichnen sich DHF-Zellen (deformed helix ferroelectric) durch einen hervorragenden Blickwinkel, schnelle Schaltzeiten, Graustufen, eine planare Optik und einen guten Kontrast aus. Ausserdem sind DHF-Zellen dank dc-freier bipolarer Ansteuerung frei von 'Ghost Effects'. Damit diese Zellen optimal funktionieren, werden ferroelektrische Mischungen mit einem sehr grossen Schaltwinkel und einer niedrigen Viskosität benötigt, um einen ausreichenden optischen Kontrast bei sehr kurzen Schaltzeiten und niedrigen Spannungen zu erzeugen. Analoge Anforderungen werden an elektroklinische elektro-optische Vorrichtungen gestellt.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase. Ausserdem ist es wichtig, dass die Komponenten untereinander gut mischbar sind, da Flüssigkristalle in der Regel als Mischungen mehrerer Komponenten zur Anwendung gelangen.

Der Erfindung liegt somit die Aufgabe zugrunde, stabile flüssigkristalline Verbindungen für DHF-Anwendungen mit niedriger Viskosität zur Verfügung zu stellen, welche in Mischungen einen grossen Schaltwinkel bewirken.

Die vorliegende Erfindung betrifft Verbindungen der allgemeinen Formel 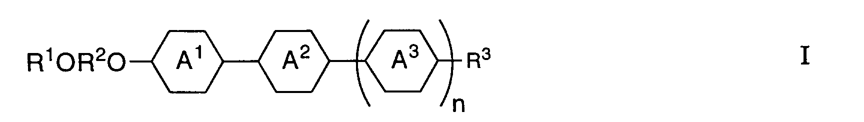 worin
- R¹: geradkettiges, unsubstituiertes oder ein- oder mehrfach mit Halogen substituiertes Alkyl oder Alkenyl mit 1 bis 8 Kohlenstoffatomen;
- R²: geradkettiges, unsubstituiertes oder ein- oder mehrfach mit Halogen substituiertes Alkylen mit 2 bis 8 Kohlenstoffatomen;
- R³: geradkettiges, unsubstituiertes oder ein- oder mehrfach mit Halogen substituiertes Alkyl, Alkoxy, Alkanoyloxy, Alkenyl, Alkenyloxy oder Alkenoyloxy mit 4 bis 12 Kohlenstoffatomen, in welchen eine oder mehrere nicht benachbarte Methylengruppen durch -O- ersetzt sein können; und
- A¹, A² und A³: unabhängig voneinander unsubstituiertes oder ein- oder mehrfach mit Halogen substituiertes 1,4-Phenylen, oder Pyridin-2,5-diyl oder Pyrimidin-2,5-diyl, und A³ auch trans-1,4-Cyclohexylen bedeuten;
und
- n: 0 oder 1 ist;
mit der Massgabe, dass mindestens einer der Ringe A¹, A² oder A³ Pyridin-2,5-diyl oder Pyrimidin-2,5-diyl bedeutet; und falls n = 0 und A² Pyrimidin-2,5-diyl ist, R³ Alkenyl Alkenyloxy, Alkanoyloxy oder Alkenoyloxy darstellt.

Es wurde überraschenderweise gefunden, dass die erfindungsgemässen Verbindungen für ferroelektrische Anwendungen und besonders in DHF-Zellen mit dc-freier Ansteuerung ausgesprochen günstige Mesophasen, grosse Schaltwinkel und kurze Schaltzeiten aufweisen.

Durch Beimischen der erfindungsgemässen Verbindungen der allgemeinen Formel I wird in Flüssigkristall-Mischungen eine bedeutende Erhöhung des S_{C}-S_{A}, S_{C}-N oder N-I Phasenüberganges bewirkt und der Schmelzpunkt signifikant gesenkt, was zu einer breiten smektischen Mesophase führt. Zudem wird die spontane Polarisation erhöht. Die erfindungsgemässen Verbindungen weisen oft breite smektische C Mesophasen mit erstaunlich niedriger Viskosität und einem sehr grossen Schaltwinkel auf. Das heisst durch Zumischen solcher Verbindungen können die Schaltzeiten von Grundmischungen erheblich gesenkt und die spontane Polarisation und der Schaltwinkel erhöht werden. Die erfindungsgemässen zwei und dreikernigen Verbindungen erweisen sich trotz ihrer nematischen oder smektischen A Phase als ausgezeichnete Komponenten für S_{C}-Mischungen, da sie den S_{C}-Phasenübergang in der Regel nicht senken. Auf diese Weise ergeben sich Phasen, die sich optimal ausrichten lassen, und die sich so ausserordentlich gut für DHF-Anwendungen mit dc-freier Ansteuerung eignen.

Der Ausdruck "geradkettiges, unsubstituiertes oder ein- oder mehrfach mit Halogen substituiertes Alkyl oder Alkenyl mit 1 bis 8 Kohlenstoffatomen" umfasst im Rahmen der vorliegenden Erfindung Gruppen wie beispielsweise Methyl, Ethyl, Propyl, Butyl, Pentyl, Hexyl, Heptyl, Octyl, Fluormethyl, Difluormethyl, Trifluormethyl, Chlor-difluormethyl, 2-Fluorethyl, 2,2-Difluorethyl, 2,2,2-Trifluorethyl, Perfluorethyl, Perfluorpropyl, 2-Propenyl, 3-Butenyl, 4-Pentenyl, 5-Hexenyl, 6-Heptenyl, 7-Octenyl, (E)-But-2-enyl, 2-E-Chlorvinyl, 2-E-Fluorvinyl, 2-E-Difluorvinyl, 2-E-Dichlorvinyl und dergleichen. Besonderes bevorzugte Gruppen haben 3 bis 6 Kohlenstoffatome und sind unsubstituiert.

Der Ausdruck "geradkettiges, unsubstituiertes oder ein- oder mehrfach mit Halogen substituiertes Alkylen mit 2 bis 8 Kohlenstoffatomen" bedeutet im Rahmen der vorliegenden Erfindung Gruppen wie beispielsweise 1,2-Ethylen, 1,3-Propylen, 1,4-Butylen, 1,5-Pentylen, 1,6-Hexylen, 1,7-Heptylen, 1,8-Octylen und dergleichen. Besonderes bevorzugte Gruppen haben 2 bis 4 Kohlenstoffatome.

Der Ausdruck "geradkettiges, unsubstituiertes oder ein- oder mehrfach mit Halogen substituiertes Alkyl, Alkoxy, Alkanoyloxy, Alkenyl, Alkenyloxy oder Alkenoyloxy mit 4 bis 12 Kohlenstoffatomen, in welchen eine oder mehrere nicht benachbarte Methylengruppen durch -O- ersetzt sein können" umfasst im Rahmen der vorliegenden Erfindung Gruppen, welche ein- oder mehrfach mit Halogen, insbesondere mit Fluor, substituiert sein können, wie beispielsweise Butyl, Pentyl, Hexyl, Heptyl, Octyl, Nonyl, Decyl, Undecyl, Dodecyl, Butyloxy, Pentyloxy, Hexyloxy, Heptyloxy, Octyloxy, Nonyloxy, Decyloxy, Undecyloxy, Dodecyloxy, Hexanoyloxy, Heptanoyloxy, Octanoyloxy, Nonanoyloxy, Decanoyloxy, Undecanoyloxy, Dodecanoyloxy, 3-Butenyl, 4-Pentenyl, 5-Hexenyl, 6-Heptenyl, 7-Octenyl, 8-Nonenyloxy, 9-Decenyl, 10-Undecenyl, 3-Butenyloxy, 4-Pentenyloxy, 5-Hexenyloxy, 6-Heptenyloxy, 7-Octenyloxy, 8-Nonenyloxy, 9-Decenyloxy, 10-Undecenyloxy, (E)-Hex-2-enyloxy, (E)-Hept-2-enyloxy, (E)-Oct-2-enyloxy, (E)-Non-2-enyloxy, (E)-Dec-2-enyloxy, (E)-Undec-2-enyloxy, (E)-Dodec-2-enyloxy, (E)-Hex-2-enoyloxy, (E)-Hept-2-enoyloxy, (E)-Oct-2-enoyloxy, (E)-Non-2-enoyloxy, (E)-Dec-2-enoyloxy, (E)-Undec-2-enoyloxy, (E)-Dodec-2-enoyloxy, (Z)-Hex-3-enoyloxy, (Z)-Hept-3-enoyloxy, (Z)-Oct-3-enoyloxy, (Z)-Non-3-enoyloxy, (Z)-Dec-3-enoyloxy, (Z)-Undec-3-enoyloxy, (Z)-Dodec-3-enoyloxy, 4-[Ethoxy]butyloxy, 4-[Propyloxy]butyloxy, 4-[Pentyloxy]butyloxy, 4-[Hexyloxy]butyloxy, 4-[3-Butenyloxy]butyloxy, 4-[4-Pentenyloxy]butyloxy, 4-[5-Hexenyloxy]butyloxy, 4-[(E)-But-2-enyloxy]butyloxy, und dergleichen. Besonderes bevorzugte Gruppen haben 2 bis 8 Kohlenstoffatome und sind unsubstituiert.

Der Ausdruck "unsubstituiertes oder ein- oder mehrfach Halogen substituiertes 1,4-Phenylen" umfasst im Rahmen der vorliegenden Erfindung 1,4-Phenylen, 2-Fluor- bzw. 3-Fluor-1,4-phenylen, 2,3-Difluor- bzw. 2,6-Difluor-1,4-phenylen und dergleichen, insbesondere jedoch 1,4-Phenylen oder 2,3-Difluor-1,4-phenylen.

Der Ausdruck "Halogen" umfasst im Rahmen der vorliegenden Erfindung Fluor, Chlor, Brom und Jod, insbesondere jedoch Fluor.

Bevorzugte Verbindungen der Formel I sind Verbindungen der allgemeinen Formeln: 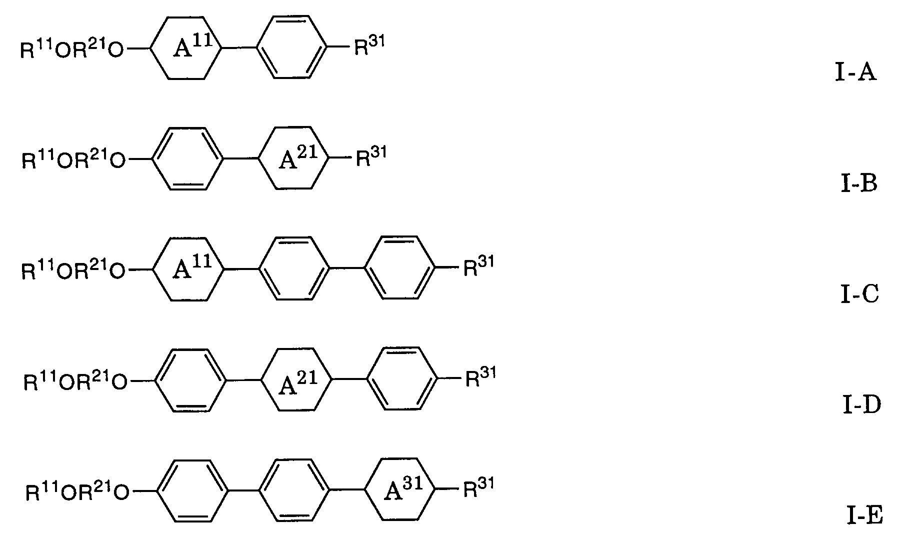 worin
- R¹¹: geradkettiges, unsubstituiertes Alkyl oder Alkenyl mit 3 bis 6 Kohlenstoffatomen;
- R²¹: geradkettiges, unsubstituiertes Alkylen mit 2 bis 4 Kohlenstoffatomen;
- A¹¹, A²¹, A³¹: Pyridin-2,5-diyl oder Pyrimidin-2,5-diyl; und
- R³¹: geradkettiges, unsubstituiertes Alkyl, Alkenyl, Alkoxy, Alkenyloxy, Alkanoyloxy, Alkenoyloxy mit 6 bis 10 Kohlenstoffatomen bedeuten.

Besonders bevorzugt sind diejenigen Verbindungen der Formeln I-A, I-B, IC und I-E, worin der Pyridin- bzw. der Pyrimidinring jeweils in 5-Stellung mit dem Rest R¹¹OR²¹O- bzw. dem Rest R³¹ verknüpft ist.

Insbesondere werden Zweiring-Verbindungen bevorzugt, so sind zum Beispiel bevorzugte Verbindungen der Formel I-A: 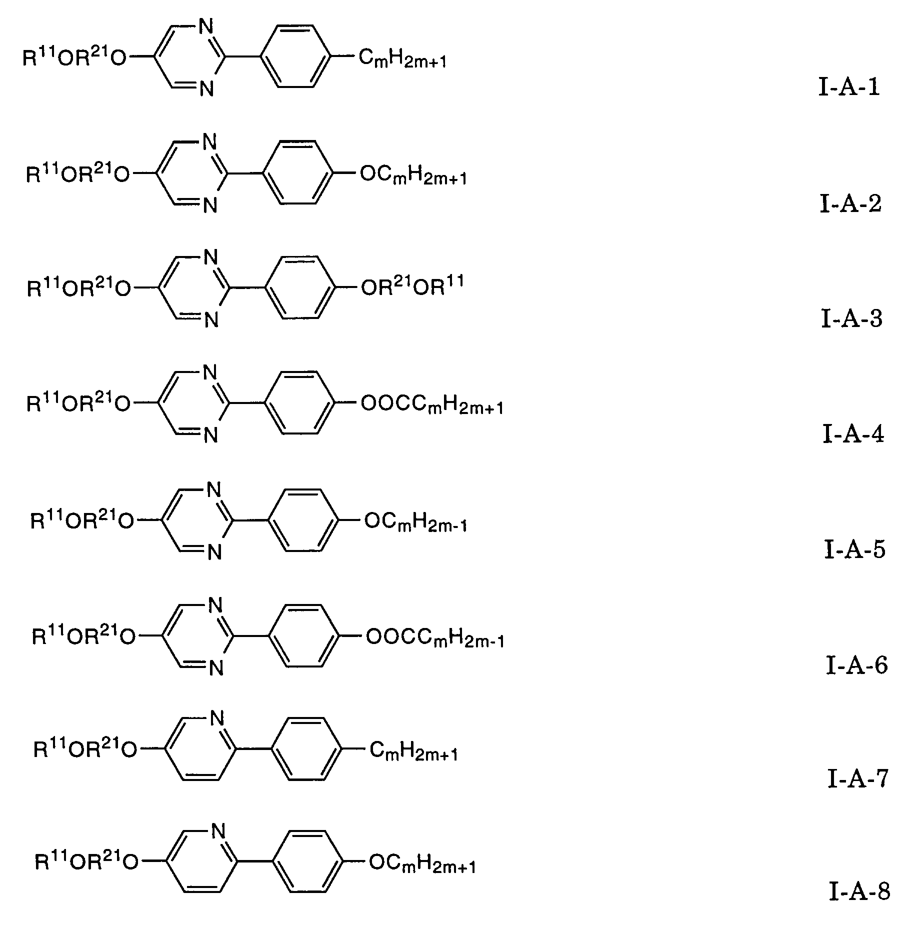 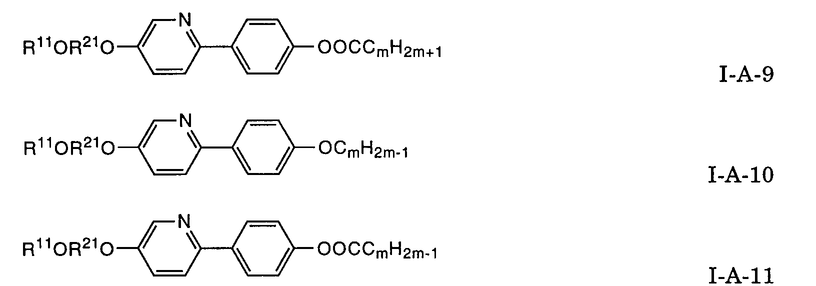 und bevorzugte Verbindungen der Formel I-B: 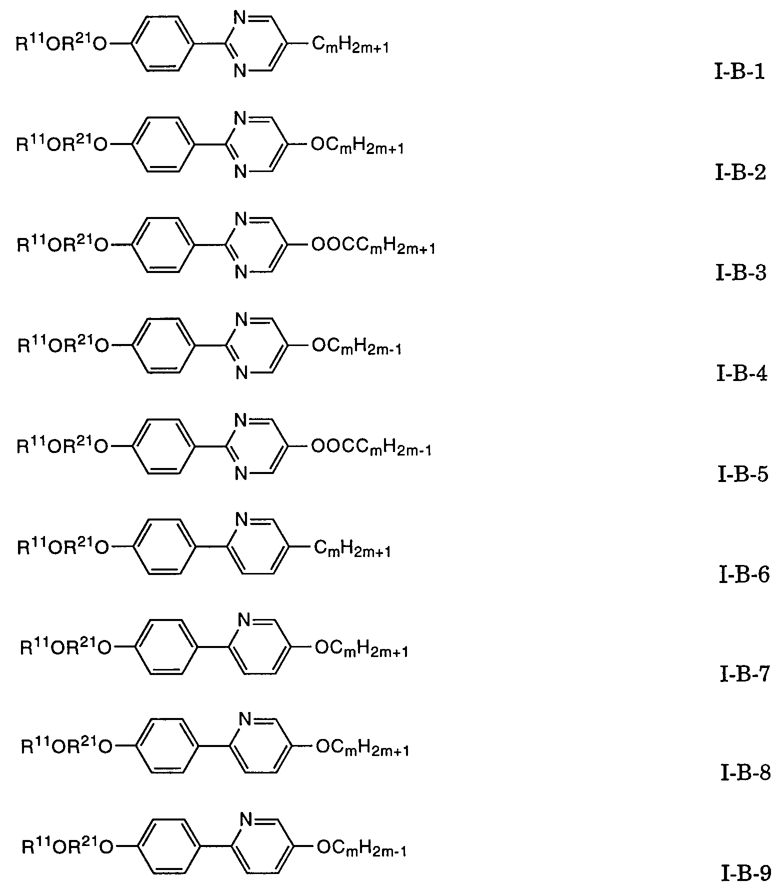 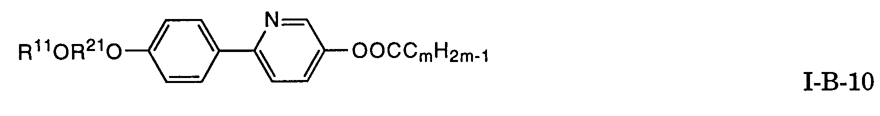 worin m eine ganze Zahl von 4 bis 8 bedeutet und den übrigen Symbolen die obengenannten Bedeutungen zukommen.

Ganz besonders bevorzugte Verbindungen der Formeln I, I-A bis I-E, I-A-1-11 und I-B-1-10 sind diejenigen, worin der Rest R² bzw. R²¹ 1,4-Butylen bedeutet.

Die erfindungsgemässen Verbindungen der allgemeinen Formel I sind synthetisch leicht zugänglich und können in an sich bekannter Weise aus entsprechend substituierten Phenolen bzw. 5-Hydroxypyridinen oder 5-Hydroxypyrimidinen und einem Alkohol, einem Alkylhalogenid oder einer Säure hergestellt werden. Die Mitsunobu-Alkylierung kann beispielsweise in Gegenwart von einer Azodicarbonsäurediethylester in Tetrahydrofuran oder DMF durchgeführt werden. Die Williams-Alkylierung kann beispielsweise in Gegenwart von einer Base wie Kaliumcarbonat in Ethylmethylketon oder DMF erfolgen. Die Veresterung kann beispielsweise in Gegenwart von N,N'-Dicyclohexylcarbodiimid (DCC) und 4-(Dimethylamino)pyridin (DMAP) in Dichlormethan oder einem anderen geeigneten Lösungsmittel wie z.B. Chloroform erfolgen. Die Ausgangsmaterialien sind bekannt und z.T. im Handel erhältlich, oder sie können wie z.B. in den nachfolgenden Schemata 1 bis 5 angegeben, hergestellt werden. Diese Reaktionen erfolgen alle in an sich bekannter Weise.

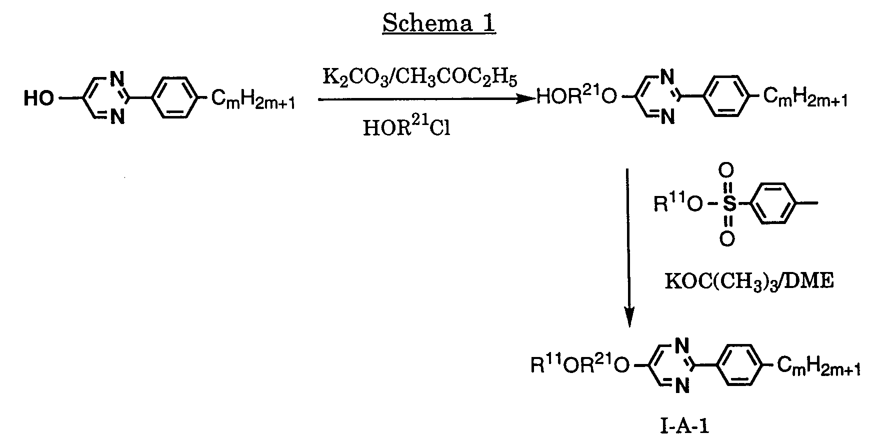

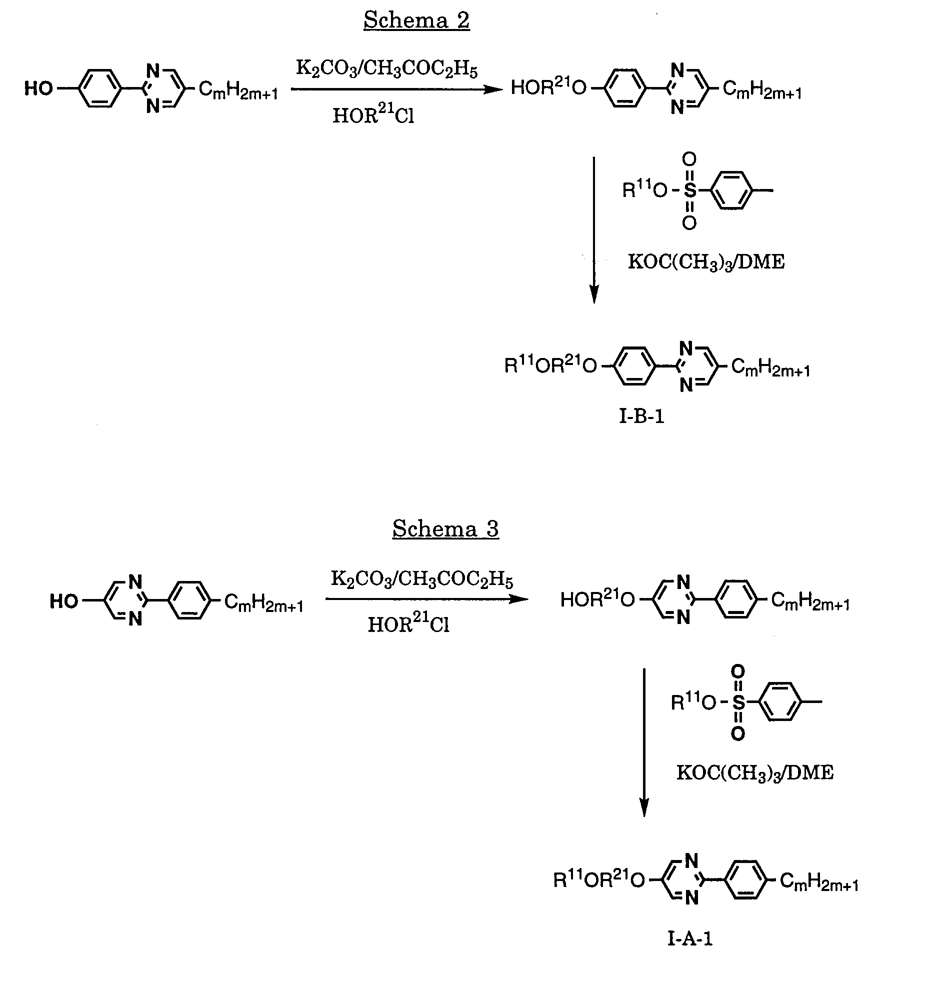

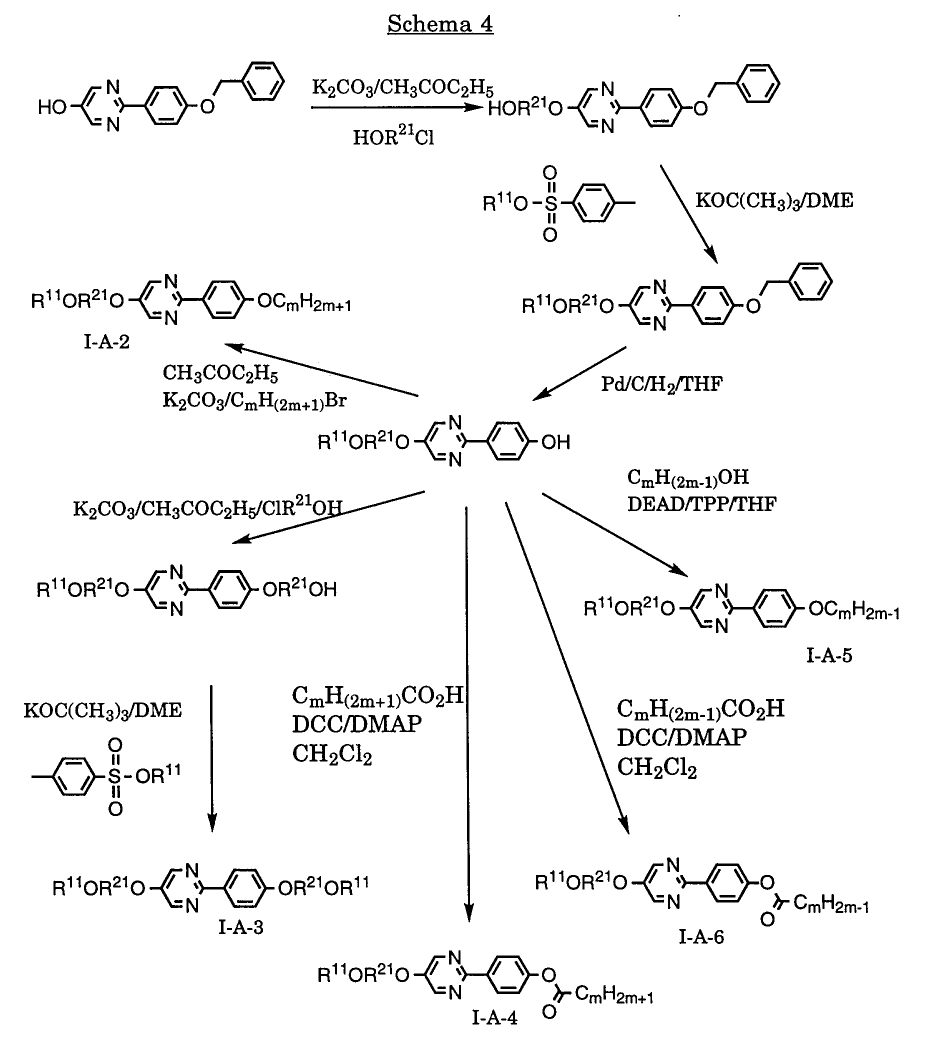

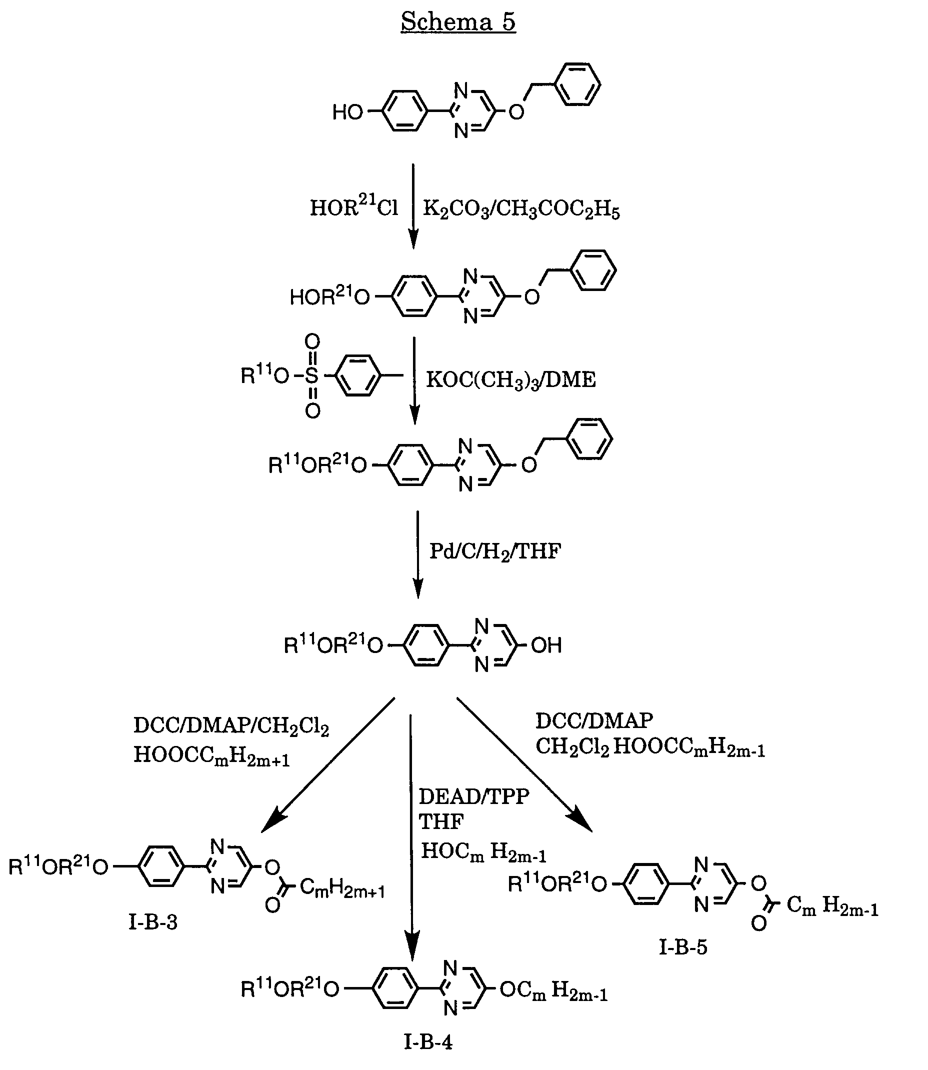

Die in den Schemata 1 - 5 verwendeten Symbole haben die obengenannten Bedeutungen.

Die Verbindungen der Formel I können in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere, bekannte flüssigkristalline Verbindungen sein.

Derartige Flüssigkristallkomponenten sind vorzugsweise achirale Verbindungen der allgemeinen Formeln 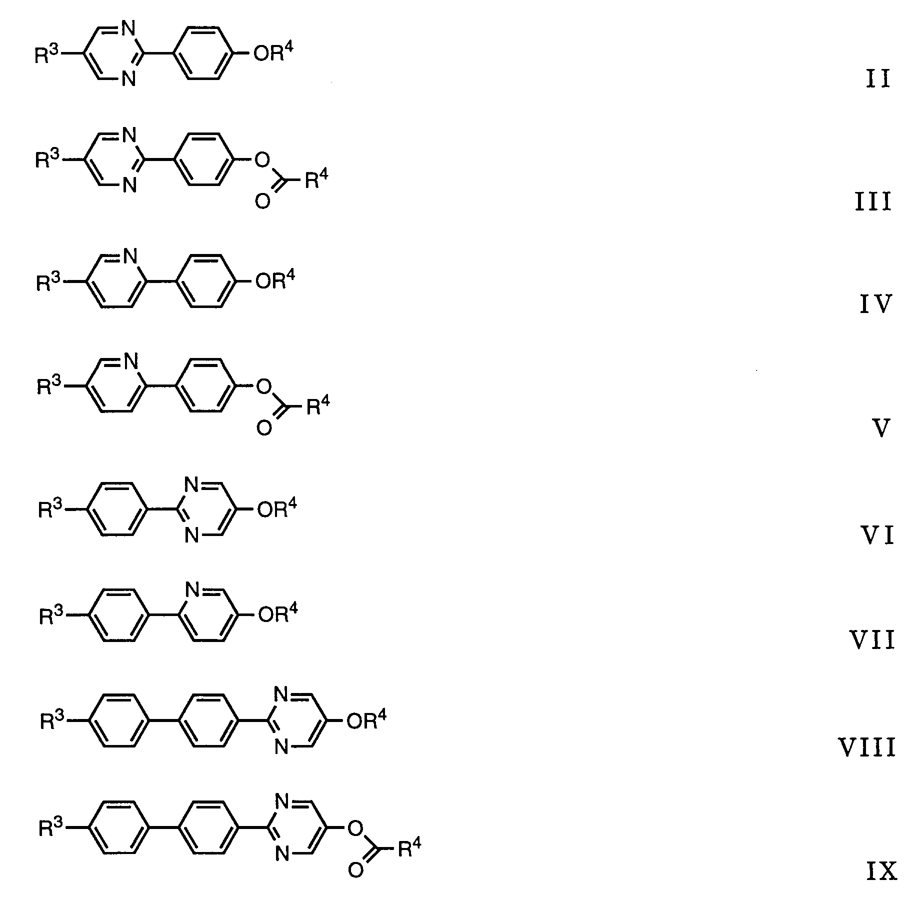 worin
- R³: Alkyl oder Alkoxy; und
- R⁴: Alkyl oder Alkenyl bedeuten.
Es muss jedoch jeweils mindestens eine chirale Komponente im Gemisch enthalten sein; d.h. es muss gegebenenfalls ein chiraler Dotierstoff zugesetzt werden. Besonders eignen sich zu diesem Zweck chirale Dotierstoffe der allgemeinen Formeln 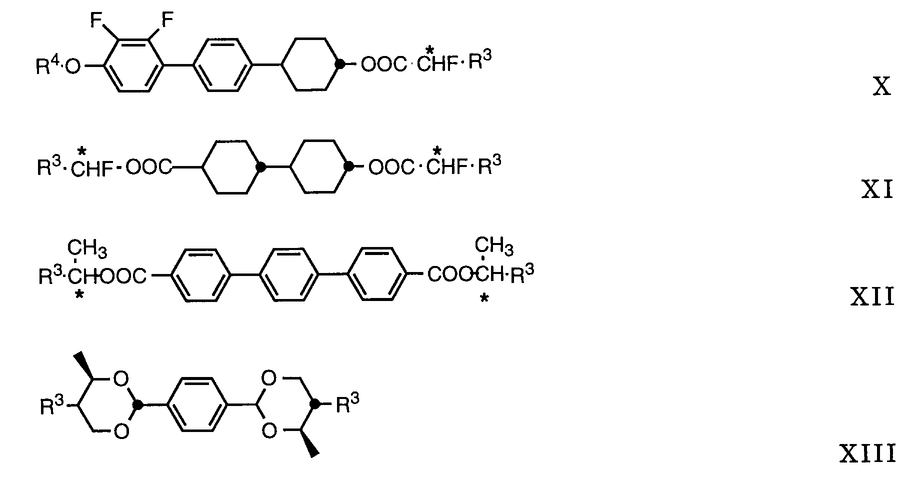 worin R³ und R⁴ die vorhergehenden Bedeutungen haben.

Der Ausdruck "Alkyl" im Zusammenhang mit den Verbindungen der Formeln II bis XIII umfasst geradkettige oder verzweigte Alkylgruppen mit 1 bis 12 Kohlenstoffatomen, vorzugsweise geradkettige Alkylgruppen mit 6 bis 12 Kohlenstoffatomen, wie Hexyl, Heptyl, Octyl, Nonyl, Decyl, Undecyl, Dodecyl.

Der Ausdruck "Alkoxy" umfasst Ethergruppen in denen der Alkylrest wie vorhergehend definiert ist.

Der Ausdruck "Alkenyl" umfasst im Zusammenhang mit den Verbindungen der Formeln II-XIII Alkenylgruppen mit 3 bis 15 Kohlenstoffatomen, wie 2E-Alkenyl, 3Z-Alkenyl, 4E-Alkenyl und Alkenyl mit endständiger Doppelbindung. Die Ausdrücke "2E-Alkenyl", "3Z-Alkenyl" und 4E-Alkenyl" umfassen vorzugsweise geradkettige Alkenylgruppen mit 3 bis 9, 4 bis 9 bzw. 5 bis 9 Kohlenstoffatomen, in welchen die Doppelbindung in 2, 3 bzw. 4 Stellung steht, wobei E und Z die Konfiguration der Doppelbindung bezeichnen. Solche Gruppen sind beispielsweise 2E-Butenyl, 2E-Pentenyl, 2E-Hexenyl, 2E-Heptenyl, 2E-Octenyl, 2E-Nonenyl, 3-Butenyl, 3Z-Pentenyl, 3Z-Hexenyl, 3Z-Heptenyl, 3Z-Octenyl, 3Z-Nonenyl, 4-Pentenyl, 4E-Hexenyl, 4E-Heptenyl, 4E-Octenyl, 4E-Nonenyl, 5-Hexenyl, 6-Heptenyl, 7-Octenyl, 8-Nonenyl und dergleichen.

Aufgrund der guten Löslichkeit der Verbindungen der Formel I und aufgrund ihrer guten Mischbarkeit untereinander kann der Anteil an Verbindungen der Formel I in den erfindungsgemässen Gemischen relativ hoch sein und bis etwa 85 Gew.-% betragen. Bevorzugt wird im allgemeinen ein Anteil von etwa 1-50, insbesondere 5-30 Gew.-% an Verbindungen der Formel I.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, Ch eine chiral nematische, S eine smektische und I die isotrope Phase; ausserdem werden folgende Abkürzungen verwendet:
- DME: Dimethoxyethan
- TPP: Triphenylphosphin.

### Beispiel 1

Eine Suspension von 0,40 g 5-(4-Hydroxybutyloxy)-2-(4-nonylphenyl)-pyrimidin und 0,76 g 4-Toluol-sulfonsäure-propylester in 25 ml 1,2-Dimethoxyethan (DME) wurde unter Stickstoffbegasung bei 0°C innert 5 Minuten mit 0,36 g Kalium-tert.-butylat versetzt. Das Reaktionsgemisch wurde über Nacht bei Raumtemperatur gerührt, auf 50 ml Wasser gegossen und dann dreimal mit je 50 ml Ethylacetat extrahiert. Die organischen Phasen wurden vereinigt, zweimal mit konzentrierter Kochsalz-Lösung gewaschen, über Magnesiumsulfat getrocknet, filtriert und das Filtrat eingeengt. Chromatographie des Rückstands an Kieselgel mit Hexan/Ethylacetat (Vol. 9:1) und Umkristallisation aus Ethylalkohol ergab 0,18 g reines 5-(4-[Propyloxy]butyloxy)-2-(4-nonylphenyl)pyrimidin; Smp. (C-S_{C}) 37°C, Phasenübergang (S_{C}-S_{A}) 45°C, Klp. (S_{A}-I) 58°C.

Das als Ausgangsmaterial verwendete 5-(4-Hydroxybutyloxy)-2-(4-nonylphenyl)pyrimidin wurde wie folgt hergestellt:
a) Eine Lösung von 1,5 g 5-Hydroxy-2-(4-nonylphenyl)pyrimidin und 2,7 g 4-Chlor-1-butanol in 25 ml Ethylmethylketon wurde mit 2,8 g fein pulverisiertem Kaliumcarbonat versetzt und das Gemisch über Nacht unter leichtem Rückfluss erwärmt. Die Suspension wurde filtriert, und das Filtrat im Vakuum eingeengt. Chromatographie des Rückstands an Kieselgel mit Hexan/Ethylacetat (Vol. 1:1) und Umkristallisation aus Ethylalkohol ergab 0,7 g 5-(4-Hydroxybutyloxy)-2-(4-nonylphenyl)pyrimidin mit Schmelzpunkt 99°C.

In analoger Weise können folgende Verbindungen hergestellt werden:
5-(4-[Methoxy]butyloxy)-2-(4-hexylphenyl)pyrimidin;
5-(4-[Ethoxy]butyloxy)-2-(4-hexylphenyl)pyrimidin;
5-(4-[Propyloxy]butyloxy)-2-(4-hexylphenyl)pyrimidin;
5-(4-[Butyloxy]butyloxy)-2-(4-hexylphenyl)pyrimidin;
5-(4-[Pentyloxy]butyloxy)-2-(4-hexylphenyl)pyrimidin;
5-(4-[Hexyloxy]butyloxy)-2-(4-hexylphenyl)pyrimidin;
5-(4-[Methoxy]butyloxy)-2-(4-heptylphenyl)pyrimidin;
5-(4-[Ethoxy]butyloxy)-2-(4-heptylphenyl)pyrimidin;
5-(4-[Propyloxy]butyloxy)-2-(4-heptylphenyl)pyrimidin;
5-(4-[Butyloxy]butyloxy)-2-(4-heptylphenyl)pyrimidin;
5-(4-[Pentyloxy]butyloxy)-2-(4-heptylphenyl)pyrimidin;
5-(4-[Hexyloxy]butyloxy)-2-(4-heptylphenyl)pyrimidin;
5-(4-[Methoxy]butyloxy)-2-(4-octylphenyl)pyrimidin;
5-(4-[Ethoxy]butyloxy)-2-(4-octylphenyl)pyrimidin;
5-(4-[Propyloxy]butyloxy)-5-(4-octylphenyl)pyrimidin;
5-(4-[Butyloxy]butyloxy)-2-(4-octylphenyl)pyrimidin;
5-(4-[Pentyloxy]butyloxy)-2-(4-octylphenyl)pyrimidin;
5-(4-[Hexyloxy]butyloxy)-2-(4-octylphenyl)pyrimidin;
5-(4-[Methoxy]butyloxy)-2-(4-nonylphenyl)pyrimidin;
5-(4-[Ethoxy]butyloxy)-2-(4-nonylphenyl)pyrimidin;
5-(4-[Butyloxy]butyloxy)-2-(4-nonylphenyl)pyrimidin;
5-(4-[Pentyloxy]butyloxy)-2-(4-nonylphenyl)pyrimidin;
5-(4-[Hexyloxy]butyloxy)-2-(4-nonylphenyl)pyrimidin;
5-(2-[Pentyloxy]ethoxy)-2-(4-nonylphenyl)pyrimidin, Smp. (C-I) 52°C, Klp. (S_{C}-I) 41°C;
5-(3-[Butyloxy]propyloxy)-2-(4-nonylphenyl)pyrimidin, Smp. (C-S_{A}) 22°C, Klp. (S_{A}-I) 32°C;
5-(5-[Ethoxy]pentyloxy)-2-(4-nonylphenyl)pyrimidin, Smp. (C-S_{A}) 53°C, Klp. (S_{A}-I) 59°C;
5-(6-[Methoxy]hexyloxy)-2-(4-nonylphenyl)pyrimidin, Smp. (C-S_{A}) 55°C, Klp. (S_{A}-I) 76°C;
2-(4-Hexylphenyl)-5-(2-[pentyloxy]ethoxy)pyridin;
2-(4-Hexylphenyl)-5-(3-[butyloxy]propyloxy)pyridin;
2-(4-Hexylphenyl)-5-(4-[propyloxy]butoxy)pyridin;
2-(4-Hexylphenyl)-5-(5-[ethoxy]pentyloxy)pyridin;
2-(4-Hexylphenyl)-5-(6-[methoxy]hexyloxy)pyridin;
2-(4-Heptylphenyl)-5-(2-[pentyloxy]ethoxy)pyridin;
2-(4-Heptylphenyl)-5-(3-[butyloxy]propyloxy)pyridin;
2-(4-Heptylphenyl)-5-(4-[propyloxy]butoxy)pyridin;
2-(4-Heptylphenyl)-5-(5-[ethoxy]pentyloxy)pyridin;
2-(4-Heptylphenyl)-5-(6-[methoxy]hexyloxy)pyridin;
2-(4-Octylphenyl)-5-(2-[pentyloxy]ethoxy)pyridin;
2-(4-Octylphenyl)-5-(3-[butyloxy]propyloxy)pyridin;
2-(4-Octylphenyl)-5-(4-[propyloxy]butoxy)pyridin;
2-(4-Octylphenyl)-5-(5-[ethoxy]pentyloxy)pyridin;
2-(4-Octylphenyl)-5-(6-[methoxy]hexyloxy)pyridin;
2-(4-Nonylphenyl)-5-(2-[pentyloxy]ethoxy)pyridin;
2-(4-Nonylphenyl)-5-(3-[butyloxy]propyloxy)pyridin;
2-(4-Nonylphenyl)-5-(4-[propyloxy]butoxy)pyridin;
2-(4-Nonylphenyl)-5-(5-[ethoxy]pentyloxy)pyridin;
2-(4-Nonylphenyl)-5-(6-[methoxy]hexyloxy)pyridin;
2-(4-Hexylphenyl)-5-(2-[hexyloxy]ethoxy)pyridin;
2-(4-Hexylphenyl)-5-(2-[heptyloxy]ethoxy)pyridin;
2-(4-Hexylphenyl)-5-(2-[octyloxy]ethoxy)pyridin;
2-(4-Heptylphenyl)-5-(2-[hexyloxy]ethoxy)pyridin;
2-(4-Heptylphenyl)-5-(2-[heptyloxy]ethoxy)pyridin;
2-(4-Heptylphenyl)-5-(2-[octyloxy]ethoxy)pyridin;
2-(4-Octylphenyl)-5-(2-[hexyloxy]ethoxy)pyridin;
2-(4-Octylphenyl)-5-(2-[heptyloxy]ethoxy)pyridin;
2-(4-Octylphenyl)-5-(2-[octyloxy]ethoxy)pyridin;
2-(4-Nonylphenyl)-5-(2-[hexyloxy]ethoxy)pyridin;
2-(4-Nonylphenyl)-5-(2-[heptyloxy]ethoxy)pyridin;
2-(4-Nonylphenyl)-5-(2-[octyloxy]ethoxy)pyridin;
2-(4-Hexylphenyl)-5-(4-[butyloxy]butyloxy)pyridin;
2-(4-Hexylphenyl)-5-(4-[pentyloxy]butyloxy)pyridin;
2-(4-Hexylphenyl)-5-(4-[hexyloxy]butyloxy)pyridin;
2-(4-Heptylphenyl)-5-(4-[butyloxy]butyloxy)pyridin;
2-(4-Heptylphenyl)-5-(4-[pentyloxy]butyloxy)pyridin;
2-(4-Heptylphenyl)-5-(4-[hexyloxy]butyloxy)pyridin;
2-(4-Octylphenyl)-5-(4-[butyloxy]butyloxy)pyridin;
2-(4-Octylphenyl)-5-(4-[pentyloxy]butyloxy)pyridin;
2-(4-Octylphenyl)-5-(4-[hexyloxy]butyloxy)pyridin;
2-(4-Nonylphenyl)-5-(4-[butyloxy]butyloxy)pyridin;
2-(4-Nonylphenyl)-5-(4-[pentyloxy]butyloxy)pyridin;
2-(4-Nonylphenyl)-5-(4-[hexyloxy]butyloxy)pyridin;
5-Hexyl-2-(4-[2-(pentyloxy)ethoxy]phenyl)pyridin;
5-Hexyl-2-(4-[3-(butyloxy)propyloxy]phenyl)pyridin;
5-Hexyl-2-(4-[4-(propyloxy)butoxy]phenyl)pyridin;
5-Hexyl-2-(4-[5-(ethoxy)pentyloxy]phenyl)pyridin;
5-Hexyl-2-(4-[6-(methoxy)hexyloxy]phenyl)pyridin;
5-Heptyl-2-(4-[2-(pentyloxy)ethoxy]phenyl)pyridin;
5-Heptyl-2-(4-[3-(butyloxy)propyloxy]phenyl)pyridin;
5-Heptyl-2-(4-[4-(propyloxy)butoxy]phenyl)pyridin;
5-Heptyl-2-(4-[5-(ethoxy)pentyloxy]phenyl)pyridin;
5-Heptyl-2-(4-[6-(methoxy)hexyloxy]phenyl)pyridin;
5-Octyl-2-(4-[2-(pentyloxy)ethoxy]phenyl)pyridin;
5-Octyl-2-(4-[3-(butyloxy)propyloxy]phenyl)pyridin;
5-Octyl-2-(4-[4-(propyloxy)butoxy]phenyl)pyridin, Smp. (C-S₄) 9°C, S₄-S₃ 12°C, S₃-S₂ 16°C, S₂-S_{C} 53°C, Klp. (S_{C}-I) 60°C;
5-Octyl-2-(4-[5-(ethoxy)pentyloxy]phenyl)pyridin;
5-Octyl-2-(4-[6-(methoxy)hexyloxy]phenyl)pyridin;
5-Nonyl-2-(4-[2-(pentyloxy)ethoxy]phenyl)pyridin;
5-Nonyl-2-(4-[3-(butyloxy)propyloxy]phenyl)pyridin;
5-Nonyl-2-(4-[5-(ethoxy)pentyloxy]phenyl)pyridin;
5-Nonyl-2-(4-[6-(methoxy)hexyloxy]phenyl)pyridin;
5-Hexyl-2-(4-[2-(butyloxy)ethoxy]phenyl)pyridin;
5-Hexyl-2-(4-[2-(pentyloxy)ethoxy]phenyl)pyridin;
5-Hexyl-2-(4-[2-(hexyloxy)ethoxy]phenyl)pyridin;
5-Hexyl-2-(4-[2-(heptyloxy)ethoxy]phenyl)pyridin;
5-Hexyl-2-(4-[2-(octyloxy)ethoxy]phenyl)pyridin;
5-Heptyl-2-(4-[2-(butyloxy)ethoxy]phenyl)pyridin;
5-Heptyl-2-(4-[2-(pentyloxy)ethoxy]phenyl)pyridin;
5-Heptyl-2-(4-[2-(hexyloxy)ethoxy]phenyl)pyridin;
5-Heptyl-2-(4-[2-(heptyloxy)ethoxy]phenyl)pyridin;
5-Heptyl-2-(4-[2-(octyloxy)ethoxy]phenyl)pyridin;
5-Octyl-2-(4-[2-(butyloxy)ethoxy]phenyl)pyridin;
5-Octyl-2-(4-[2-(pentyloxy)ethoxy]phenyl)pyridin;
5-Octyl-2-(4-[2-(hexyloxy)ethoxy]phenyl)pyridin;
5-Octyl-2-(4-[2-(heptyloxy)ethoxy]phenyl)pyridin;
5-Octyl-2-(4-[2-(octyloxy)ethoxy]phenyl)pyridin;
5-Nonyl-2-(4-[2-(butyloxy)ethoxy]phenyl)pyridin;
5-Nonyl-2-(4-[2-(pentyloxy)ethoxy]phenyl)pyridin;
5-Nonyl-2-(4-[2-(hexyloxy)ethoxy]phenyl)pyridin;
5-Nonyl-2-(4-[2-(heptyloxy)ethoxy]phenyl)pyridin;
5-Nonyl-2-(4-[2-(octyloxy)ethoxy]phenyl)pyridin;
5-Hexyl-2-(4-[4-(butyloxy)butoxy]phenyl)pyridin;
5-Hexyl-2-(4-[4-(pentyloxy)butoxy]phenyl)pyridin;
5-Hexyl-2-(4-[4-(hexyloxy)butoxy]phenyl)pyridin;
5-Heptyl-2-(4-[4-(butyloxy)butoxy]phenyl)pyridin;
5-Heptyl-2-(4-[4-(pentyloxy)butoxy]phenyl)pyridin;
5-Heptyl-2-(4-[4-(hexyloxy)butoxy]phenyl)pyridin;
5-Octyl-2-(4-[4-(butyloxy)butoxy]phenyl)pyridin;
5-Octyl-2-(4-[4-(pentyloxy)butoxy]phenyl)pyridin;
5-Octyl-2-(4-[4-(hexyloxy)butoxy]phenyl)pyridin;
5-Nonyl-2-(4-[4-(butyloxy)butoxy]phenyl)pyridin;
5-Nonyl-2-(4-[4-(pentyloxy)butoxy]phenyl)pyridin;
5-Nonyl-2-(4-[4-(hexyloxy)butoxy]phenyl)pyridin;
5-(4-[Methoxy]butyloxy)-2-(4-pentyl-4'-biphenyl)pyrimidin;
5-(4-[Ethoxy]butyloxy)-2-(4-pentyl-4'-biphenyl)pyrimidin;
5-(4-[Propoxy]butyloxy)-2-(4-pentyl-4'-biphenyl)pyrimidin;
5-(4-[Butyloxy]butyloxy)-2-(4-pentyl-4'-biphenyl)pyrimidin;
5-Pentyl-2-(4-(4-[Methoxy]butyloxy)-4'-biphenyl)pyrimidin;
5-Pentyl-2-(4-(4-[Ethoxy]butyloxy)-4'-biphenyl)pyrimidin;
5-Pentyl-2-(4-(4-[Propyloxy]butyloxy)-4'-biphenyl)pyrimidin;
5-Pentyl-2-(4-(4-[Butyloxy]butyloxy)-4'-biphenyl)pyrimidin;
5-(4-Pentylphenyl)-2-(4-[4-(methoxy)butyloxy]phenyl)pyrimidin;
5-(4-Pentylphenyl)-2-(4-[4-(ethoxy)butyloxy]phenyl)pyrimidin;
5-(4-Pentylphenyl)-2-(4-[4-(propyloxy)butyloxy]phenyl)pyrimidin;
5-(4-Pentylphenyl)-2-(4-[4-(butyloxy)butyloxy]phenyl)pyrimidin;
2-(4-Pentylphenyl)-5-(4-[4-(Methoxy)butyloxy]phenyl)pyrimidin;
2-(4-Pentylphenyl)-5-(4-[4-(ethoxy)butyloxy]phenyl)pyrimidin;
2-(4-Pentylphenyl)-5-(4-[4-(propyloxy)butyloxy]phenyl)pyrimidin;
2-(4-Pentylphenyl)-5-(4-[4-(butyloxy)butyloxy]phenyl)pyrimidin;
5-(4-Pentylphenyl)-2-(4-[4-(butyloxy)butyloxy]phenyl)pyridin;
2-(4-Pentylphenyl)-5-(4-[4-(butyloxy)butyloxy]phenyl)pyridin;
5-Pentyl-2-(4-(4-[Butyloxy]butyloxy)-4'-biphenyl)pyridin;
5-(4-[Butyloxy]butyloxy)-2-(4-pentyl-4'-biphenyl)pyridin.

### Beispiel 2

Eine Lösung von 0,2 g 4-(5-[4-(Propyloxy)butyloxy]pyrimidin-2-yl)phenol und 0,15 g 1-Bromoctan in 25 ml Ethylmethylketon wurde mit 0,4 g fein pulverisiertem Kaliumcarbonat versetzt und das Gemisch über Nacht unter leichtem Rückfluss erwärmt. Die Suspension wurde filtriert, und das Filtrat im Vakuum eingeengt. Chromatographie des Rückstands an Kieselgel mit Hexan/Ethylacetat (Vol. 9:1) ergibt 0,14 g 2-(4-Octyloxyphenyl)-5-(4-[propyloxy]butyloxy)pyrimidin; Smp. (C-N) 76°C, Phasenübergang (S_{C}-N) 70°C, Klp. (N-I) 77°C.

Das als Ausgangsmaterial verwendete 4-(5-[4-(Propyloxy)butyloxy]-pyrimidin-2-yl)phenol wurde wie folgt hergestellt:
a) Eine Lösung von 5,0 g 5-Hydroxy-2-(4-Benzyloxyphenyl)pyrimidin und 2,3 g 4-Chlor-1-butanol in 100 ml Ethylmethylketon wurde mit 9,9 g fein pulverisiertem Kaliumcarbonat versetzt und das Gemisch über Nacht unter leichtem Rückfluss erwärmt. Die Suspension wurde filtriert, und das Filtrat im Vakuum eingeengt. Chromatographie des Rückstands an Kieselgel mit Hexan/Ethylacetat (Vol. 1:1) ergab 1,6 g 2-(4-Benzyloxyphenyl)-5-(4-hydroxybutyloxy)pyrimidin mit Smp. 114°C.
b) Eine Suspension von 1,2 g 2-(4-Benzyloxyphenyl)-5-(4-hydroxybutyloxy)-pyrimidin und 2,4 g 4-Toluol-sulfonsäure-propylester in 25 ml 1,2-Dimethoxyethan wurde unter Stickstoffbegasung bei 0°C innert 5 Minuten mit 1,2 g Kalium-tert.-butylat versetzt. Das Reaktionsgemisch wurde über Nacht bei Raumtemperatur gerührt, auf 50 ml Wasser gegossen und dann dreimal mit je 50 ml Ethylacetat extrahiert. Die organischen Phasen wurden vereinigt, zweimal mit konzentrierter Kochsalz-Lösung gewaschen, über Magnesiumsulfat getrocknet, filtriert und das Filtrat eingeengt. Chromatographie des Rückstands an Kieselgel mit Hexan/Ethylacetat (Vol. 1:1) und Umkristallisation aus Ethylacetat ergab 0,8 g reines 2-(4-Benzyloxyphenyl)-5-(4-[propyloxy]butyloxy)pyrimidin mit Smp. 115°C.
c) Ein Gemisch aus 0,8 g 2-(4-Benzyloxyphenyl)-5-(4-propyloxybutyloxy)-pyrimidin, 2 Tropfen Essigsäure und 50 ml Ethylacetat wurde mit 0,3 g Palladium auf Aktivkohle (10%) versetzt und bei Normaldruck und Raumtemperatur bis zum Stillstand der Wasserstoffaufnahme hydriert. Das anorganische Material wurde über Sillit filtriert und das Filtrat eingeengt. Umkristallisation des Rohproduktes aus Hexan ergab 0,6 g reines 4-(5-[4-(Propyloxy)butyloxy]pyrimidin-2-yl)phenol mit Smp. 103°C.

In analoger Weise können folgende Verbindungen hergestellt werden:
2-(4-[Hexyloxy]phenyl)-5-(4-propyloxybutyloxy)pyrimidin;
2-(4-[Heptyloxy]phenyl)-5-(4-propyloxybutyloxy)pyrimidin;
2-(4-[Nonyloxy]phenyl)-5-(4-propyloxybutyloxy)pyrimidin, Smp. (C-S_{C}) 67°C, S_{C}-N 70°C, Klp. (N-I) 77°C;
2-(4-[Decyloxy]phenyl)-5-(4-[propyloxy]butyloxy)pyrimidin, Smp. (C-N) 72°C, S_{C}-N, 71°C, Klp. (N-I) 78°C;
2-(4-[Undecyloxy]phenyl)-5-(4-[propyloxy]butyloxy)pyrimidin, Smp. (C-S_{C}) 64°C, S_{C}-N, 72°C, Klp. (N-I) 76°C;
2-(4-[Dodecyloxy]phenyl)-5-(4-[propyloxy]butyloxy)pyrimidin, Smp. (C-S_{C}) 70°C, S_{C}-N, 72°C, Klp. (N-I) 77°C;
2-(4-[Hexyloxy]phenyl)-5-(4-[butyloxy]butyloxy)pyrimidin;
2-(4-[Heptyloxy]phenyl)-5-(4-[butyloxy]butyloxy)pyrimidin;
2-(4-[Nonyloxy]phenyl)-5-(4-[butyloxy]butyloxy)pyrimidin;
2-(4-[Decyloxy]phenyl)-5-(4-[butyloxy]butyloxy)pyrimidin;
2-(4-[Undecyloxy]phenyl)-5-(4-[butyloxy]butyloxy)pyrimidin;
2-(4-[Dodecyloxy]phenyl)-5-(4-[butyloxy]butyloxy)pyrimidin;
2-(4-[Hexyloxy]phenyl)-5-(4-[pentyloxy]butyloxy)pyrimidin;
2-(4-[Heptyloxy]phenyl)-5-(4-[pentyloxy]butyloxy)pyrimidin;
2-(4-[Nonyloxy]phenyl)-5-(4-[pentyloxy]butyloxy)pyrimidin;
2-(4-[Decyloxy]phenyl)-5-(4-[pentyloxy]butyloxy)pyrimidin;
2-(4-[Undecyloxy]phenyl)-5-(4-[pentyloxy]butyloxy)pyrimidin;
2-(4-[Dodecyloxy]phenyl)-5-(4-[pentyloxy]butyloxy)pyrimidin;
2-(4-[Hexyloxy]phenyl)-5-(4-[hexyloxy]butyloxy)pyrimidin;
2-(4-[Heptyloxy]phenyl)-5-(4-[hexyloxy]butyloxy)pyrimidin;
2-(4-[Nonyloxy]phenyl)-5-(4-[hexyloxy]butyloxy)pyrimidin;
2-(4-[Decyloxy]phenyl)-5-(4-[hexyloxy]butyloxy)pyrimidin;
2-(4-[Undecyloxy]phenyl)-5-(4-[hexyloxy]butyloxy)pyrimidin;
2-(4-[Dodecyloxy]phenyl)-5-(4-[hexyloxy]butyloxy)pyrimidin;
2-(4-[Hexyloxy]phenyl)-5-(2-[pentyloxy]ethoxy)pyrimidin;
2-(4-[Heptyloxy]phenyl)-5-(2-[pentyloxy]ethoxy)pyrimidin;
2-(4-[Octyloxy]phenyl)-5-(2-[pentyloxy]ethoxy)pyrimidin;
2-(4-[Nonyloxy]phenyl)-5-(2-[pentyloxy]ethoxy)pyrimidin;
2-(4-[Decyloxy]phenyl)-5-(2-[pentyloxy]ethoxy)pyrimidin;
2-(4-[Undecyloxy]phenyl)-5-(2-[pentyloxy]ethoxy)pyrimidin;
2-(4-[Dodecyloxy]phenyl)-5-(2-[pentyloxy]ethoxy)pyrimidin;
2-(4-[Hexyloxy]phenyl)-5-(2-[hexyloxy]ethoxy)pyrimidin;
2-(4-[Heptyloxy]phenyl)-5-(2-[hexyloxy]ethoxy)pyrimidin;
2-(4-[Octyloxy]phenyl)-5-(2-[hexyloxy]ethoxy)pyrimidin;
2-(4-[Nonyloxy]phenyl)-5-(2-[hexyloxy]ethoxy)pyrimidin;
2-(4-[Decyloxy]phenyl)-5-(2-[hexyloxy]ethoxy)pyrimidin;
2-(4-[Undecyloxy]phenyl)-5-(2-[hexyloxy]ethoxy)pyrimidin;
2-(4-[Dodecyloxy]phenyl)-5-(2-[hexyloxy]ethoxy)pyrimidin;
2-(4-[Hexyloxy]phenyl)-5-(2-[heptyloxy]ethoxy)pyrimidin;
2-(4-[Heptyloxy]phenyl)-5-(2-[heptyloxy]ethoxy)pyrimidin;
2-(4-[Octyloxy]phenyl)-5-(2-[heptyloxy]ethoxy)pyrimidin;
2-(4-[Nonyloxy]phenyl)-5-(2-[heptyloxy]ethoxy)pyrimidin;
2-(4-[Decyloxy]phenyl)-5-(2-[heptyloxy]ethoxy)pyrimidin;
2-(4-[Undecyloxy]phenyl)-5-(2-[heptyloxy]ethoxy)pyrimidin;
2-(4-[Dodecyloxy]phenyl)-5-(2-[heptyloxy]ethoxy)pyrimidin;
2-(4-[Hexyloxy]phenyl)-5-(6-[methoxy]hexyloxy)pyrimidin;
2-(4-[Heptyloxy]phenyl)-5-(6-[methoxy]hexyloxy)pyrimidin;
2-(4-[Octyloxy]phenyl)-5-(6-[methoxy]hexyloxy)pyrimidin;
2-(4-[Nonyloxy]phenyl)-5-(6-[methoxy]hexyloxy)pyrimidin;
2-(4-[Decyloxy]phenyl)-5-(6-[methoxy]hexyloxy)pyrimidin;
2-(4-[Undecyloxy]phenyl)-5-(6-[methoxy]hexyloxy)pyrimidin;
2-(4-[Dodecyloxy]phenyl)-5-(6-[methoxy]hexyloxy)pyrimidin;
2-(4-[Hexyloxy]phenyl)-5-(6-[ethoxy]hexyloxy)pyrimidin;
2-(4-[Heptyloxy]phenyl)-5-(6-[ethoxy]hexyloxy)pyrimidin;
2-(4-[Octyloxy]phenyl)-5-(6-[ethoxy]hexyloxy)pyrimidin;
2-(4-[Nonyloxy]phenyl)-5-(6-[ethoxy]hexyloxy)pyrimidin;
2-(4-[Decyloxy]phenyl)-5-(6-[ethoxy]hexyloxy)pyrimidin;
2-(4-[Undecyloxy]phenyl)-5-(6-[ethoxy]hexyloxy)pyrimidin;
2-(4-[Dodecyloxy]phenyl)-5-(6-[ethoxy]hexyloxy)pyrimidin;
2-(4-[Hexyloxy]phenyl)-5-(6-[propyloxy]hexyloxy)pyrimidin;
2-(4-[Heptyloxy]phenyl)-5-(6-[propyloxy]hexyloxy)pyrimidin;
2-(4-[Octyloxy]phenyl)-5-(6-[propyloxy]hexyloxy)pyrimidin;
2-(4-[Nonyloxy]phenyl)-5-(6-[propyloxy]hexyloxy)pyrimidin;
2-(4-[Decyloxy]phenyl)-5-(6-[propyloxy]hexyloxy)pyrimidin;
2-(4-[Undecyloxy]phenyl)-5-(6-[propyloxy]hexyloxy)pyrimidin;
2-(4-[Dodecyloxy]phenyl)-5-(6-[propyloxy]hexyloxy)pyrimidin;
2-(4-[Hexyloxy]phenyl)-5-(6-[butyloxy]hexyloxy)pyrimidin;
2-(4-[Heptyloxy]phenyl)-5-(6-[butyloxy]hexyloxy)pyrimidin;
2-(4-[Octyloxy]phenyl)-5-(6-[butyloxy]hexyloxy)pyrimidin;
2-(4-[Nonyloxy]phenyl)-5-(6-[butyloxy]hexyloxy)pyrimidin;
2-(4-[Decyloxylphenyl)-5-(6-[butyloxy]hexyloxy)pyrimidin;
2-(4-[Undecyloxy]phenyl)-5-(6-[butyloxy]hexyloxy)pyrimidin;
2-(4-[Dodecyloxy]phenyl)-5-(6-[butyloxy]hexyloxy)pyrimidin.

### Beispiel 3

Eine Suspension von 0,1 g 2-(4-[4-Hydroxybutyloxy]phenyl)-5-(4-[propyloxy]butyloxy)pyrimidin und 0,19 g 4-Toluol-sulfonsäure-propylester in 25 ml 1, 2-Dimethoxyethan wurde unter Stickstoffbegasung bei 0°C innert 5 Minuten mit 0,09 g Kalium-tert.-butylat versetzt. Das Reaktionsgemisch wurde über Nacht bei Raumtemperatur gerührt, auf 50 ml Wasser gegossen und dann dreimal mit je 50 ml Ethylacetat extrahiert. Die organischen Phasen wurden vereinigt, zweimal mit konzentrierter Kochsalz-Lösung gewaschen, über Magnesiumsulfat getrocknet, filtriert und das Filtrat eingeengt. Chromatographie des Rückstands an Kieselgel mit Hexan/Ethylacetat (Vol. 9:1) und Umkristallisation aus Ethylalkohol ergab 0,04 g reines 2-(4-[(Propyloxy)butyloxy]phenyl)-5-(4-[propyloxy]butyloxy)pyrimidin; Smp. (C-I) 82°C.

Das als Ausgangsmaterial verwendete 2-(4-[4-Hydroxybutyloxy]phenyl)-5-(4-[propyloxy]butyloxy)pyrimidin wurde wie folgt hergestellt:
a) Eine Lösung von 0,2 g 4-(5-[4-(Propyloxy)butyloxy]pyrimidin-2-yl)phenol und 0,36 g 4-Chlor-1-butanol in 25 ml Ethylmethylketon wurde mit 0,37 g fein pulverisiertem Kaliumcarbonat versetzt und das Gemisch über Nacht unter leichtem Rückfluss erwärmt. Die Suspension wurde filtriert, und das Filtrat im Vakuum eingeengt. Chromatographie des Rückstands an Kieselgel mit Hexan/Ethylacetat (Vol. 1:1) und Umkristallisation aus Ethylalkohol ergab 0,1 g 2-(4-[4-Hydroxybutyloxy]phenyl)-5-(4-[propyloxy]butyloxy)pyrimidin.

In analoger Weise können folgende Verbindungen hergestellt werden:
2-(4-[(Pentyloxy)butyloxy]phenyl)-5-(4-[propyloxy]butyloxy)pyrimidin;
2-(4-[(Heptyloxy)butyloxy]phenyl)-5-(4-[propyloxy]butyloxy)pyrimidin;
2-(4-[(Nonyloxy)butyloxy]phenyl)-5-(4-[propyloxy]butyloxy)pyrimidin;
2-(4-[(Propyloxy)butyloxy]phenyl)-5-(4-[pentyloxy]butyloxy)pyrimidin;
2-(4-[(Propyloxy)butyloxy]phenyl)-5-(4-[heptyloxy]butyloxy)pyrimidin;
2-(4-[(Propyloxy)butyloxy]phenyl)-5-(4-[nonyloxy]butyloxy)pyrimidin;
2-(6-[(Methoxy)hexyloxy]phenyl)-5-(4-[propyloxy]butyloxy)pyrimidin;
2-(6-[(Ethoxy)hexyloxy]phenyl)-5-(4-[propyloxy]butyloxy)pyrimidin;
2-(6-[(Propyloxy)hexyloxy]phenyl)-5-(4-[propyloxy]butyloxy)pyrimidin;
2-(6-[(Butyloxy)hexyloxy]phenyl)-5-(4-[propyloxy]butyloxy)pyrimidin;
2-(4-[(Propyloxy)butyloxy]phenyl)-5-(6-[methoxy]hexyloxy)pyrimidin;
2-(4-[(Propyloxy)butyloxy]phenyl)-5-(6-[ethoxy]hexyloxy)pyrimidin;
2-(4-[(Propyloxy)butyloxy]phenyl)-5-(6-[propyloxy]hexyloxy)pyrimidin;
2-(4-[(Propyloxy)butyloxy]phenyl)-5-(6-[butyloxy]hexyloxy)pyrimidin;
2-(2-[(Propyloxy)ethoxy]phenyl)-5-(2-[propyloxy]ethoxy)pyrimidin;
2-(2-[(Butyloxy)ethoxy]phenyl)-5-(2-[butyloxy]ethoxy)pyrimidin;
2-(2-[(Pentyloxy)ethoxy]phenyl)-5-(2-[pentyloxy]ethoxy)pyrimidin;
2-(2-[(Hexyloxy)ethoxy]phenyl)-5-(2-[hexyloxy]ethoxy)pyrimidin;
2-(2-[(Heptyloxy)ethoxy]phenyl)-5-(2-[heptyloxy]ethoxy)pyrimidin;
2-(2-[(Octyloxy)ethoxy]phenyl)-5-(2-[octyloxy]ethoxy)pyrimidin;
2-(6-[(Methoxy)hexyloxy]phenyl)-5-(6-[methoxy]hexyloxy)pyrimidin;
2-(6-[(Ethoxy)hexyloxy]phenyl)-5-(6-[ethoxy]hexyloxy)pyrimidin;
2-(6-[(Propyloxy)hexyloxy]phenyl)-5-(6-[propyloxy]hexyloxy)pyrimidin;
2-(6-[(Butyloxy)hexyloxy]phenyl)-5-(6-[butyloxy]hexyloxy)pyrimidin.

### Beispiel 4

Zu einer Lösung von 0,2 g 4-(5-[4-(Propyloxy)butyloxy] pyrimidin-2-yl)phenol, 0,1 g Octansäure und 0,05 g 4-(Dimethylamino)pyridin in 25 ml Dichlormethan wurde unter Rühren innert 5 Minuten 0,16 g N,N'-Dicyclohexylcarbodiimid gegeben. Das Reaktionsgemisch wurde über Nacht weiter gerührt, filtriert und dann eingeengt. Chromatographie des Rückstandes an Kieselgel mit Hexan/Ethylacetat (Vol. 9:1) und Umkristallisation der gemäss Dünnschichtchromatographie reinen Fraktionen aus Ethylalkohol ergab 0,08 g 4-(5-[4-(Propyloxy)butyloxy]-pyrimidin-2-yl)phenyl-octanoat; Smp. (C-N) 62°C, Phasenübergang (S_{C}-N) 61°C, Klp. (N-I) 63°C.

In analoger Weise können folgende Verbindungen hergestellt werden:
4-(5-[4-(Propyloxy)butyloxy]pyrimidin-2-yl)phenyl-hexanoat;
4-(5-[4-(Propyloxy)butyloxy]pyrimidin-2-yl)phenyl-heptanoat;
4-(5-[4-(Propyloxy)butyloxy]pyrimidin-2-yl)phenyl-nonanoat;
4-(5-[4-(Propyloxy)butyloxy]pyrimidin-2-yl)phenyl-decanoat;
4-(5-[4-(Propyloxy)butyloxy]pyrimidin-2-yl)phenyl-undecanoat;
4-(5-[4-(Propyloxy)butyloxy]pyrimidin-2-yl)phenyl-dodecanoat;
4-(5-[4-(Butyloxy)butyloxy]pyrimidin-2-yl)phenyl-hexanoat;
4-(5-[4-(Butyloxy)butyloxy]pyrimidin-2-yl)phenyl-heptanoat;
4-(5-[4-(Butyloxy)butyloxy]pyrimidin-2-yl)phenyl-nonanoat;
4-(5-[4-(Butyloxy)butyloxy]pyrimidin-2-yl)phenyl-decanoat;
4-(5-[4-(Butyloxy)butyloxy]pyrimidin-2-yl)phenyl-undecanoat;
4-(5-[4-(Butyloxy)butyloxy]pyrimidin-2-yl)phenyl-dodecanoat;
4-(5-[4-(Pentyloxy)butyloxy]pyrimidin-2-yl)phenyl-hexanoat;
4-(5-[4-(Pentyloxy)butyloxy]pyrimidin-2-yl)phenyl-heptanoat;
4-(5-[4-(Pentyloxy)butyloxy]pyrimidin-2-yl)phenyl-nonanoat;
4-(5-[4-(Pentyloxy)butyloxy]pyrimidin-2-yl)phenyl-decanoat;
4-(5-[4-(Pentyloxy)butyloxy]pyrimidin-2-yl)phenyl-undecanoat;
4-(5-[4-(Pentyloxy)butyloxy]pyrimidin-2-yl)phenyl-dodecanoat;
4-(5-[4-(Hexyloxy)butyloxy]pyrimidin-2-yl)phenyl-hexanoat;
4-(5-[4-(Hexyloxy)butyloxy]pyrimidin-2-yl)phenyl-heptanoat;
4-(5-[4-(Hexyloxy)butyloxy]pyrimidin-2-yl)phenyl-nonanoat;
4-(5-[4-(Hexyloxy)butyloxy]pyrimidin-2-yl)phenyl-decanoat;
4-(5-[4-(Hexyloxy)butyloxy]pyrimidin-2-yl)phenyl-undecanoat;
4-(5-[4-(Hexyloxy)butyloxy]pyrimidin-2-yl)phenyl-dodecanoat;
4-(5-[4-(Propyloxy)butyloxy]pyrimidin-2-yl)phenyl-(E)-hex-2-enoat;
4-(5-[4-(Propyloxy)butyloxy]pyrimidin-2-yl)phenyl-(E)-hept-2-enoat;
4-(5-[4-(Propyloxy)butyloxy]pyrimidin-2-yl)phenyl-(E)-oct-2-enoat;
4-(5-[4-(Propyloxy)butyloxy]pyrimidin-2-yl)phenyl-(E)-non-2-enoat;
4-(5-[4-(Propyloxy)butyloxy]pyrimidin-2-yl)phenyl-(E)-dec-2-enoat;
4-(5-[4-(Propyloxy)butyloxy]pyrimidin-2-yl)phenyl-(E)-undec-2-enoat;
4-(5-[4-(Propyloxy)butyloxy]pyrimidin-2-yl)phenyl-(E)-dodec-2-enoat;
4-(5-[4-(Butyloxy)butyloxy]pyrimidin-2-yl)phenyl-(E)-hex-2-enoat;
4-(5-[4-(Butyloxy)butyloxy]pyrimidin-2-yl)phenyl-(E)-hept-2-enoat;
4-(5-[4-(Butyloxy)butyloxy]pyrimidin-2-yl)phenyl-(E)-oct-2-enoat;
4-(5-[4-(Butyloxy)butyloxy]pyrimidin-2-yl)phenyl-(E)-non-2-enoat;
4-(5-[4-(Butyloxy)butyloxy]pyrimidin-2-yl)phenyl-(E)-dec-2-enoat;
4-(5-[4-(Butyloxy)butyloxy]pyrimidin-2-yl)phenyl-(E)-undec-2-enoat;
4-(5-[4-(Butyloxy)butyloxy]pyrimidin-2-yl)phenyl-(E)-dodec-2-enoat;
4-(5-[4-(Pentyloxy)butyloxy]pyrimidin-2-yl)phenyl-(E)-hex-2-enoat;
4-(5-[4-(Pentyloxy)butyloxy]pyrimidin-2-yl)phenyl-(E)-hept-2-enoat;
4-(5-[4-(Pentyloxy)butyloxy]pyrimidin-2-yl)phenyl-(E)-oct-2-enoat;
4-(5-[4-(Pentyloxy)butyloxy]pyrimidin-2-yl)phenyl-(E)-non-2-enoat;
4-(5-[4-(Pentyloxy)butyloxy]pyrimidin-2-yl)phenyl-(E)-dec-2-enoat;
4-(5-[4-(Pentyloxy)butyloxy]pyrimidin-2-yl)phenyl-(E)-undec-2-enoat;
4-(5-[4-(Pentyloxy)butyloxy]pyrimidin-2-yl)phenyl-(E)-dodec-2-enoat;
4-(5-[4-(Hexyloxy)butyloxy]pyrimidin-2-yl)phenyl-(E)-hex-2-enoat;
4-(5-[4-(Hexyloxy)butyloxy]pyrimidin-2-yl)phenyl-(E)-hept-2-enoat;
4-(5-[4-(Hexyloxy)butyloxylpyrimidin-2-yl)phenyl-(E)-oct-2-enoat;
4-(5-[4-(Hexyloxy)butyloxy]pyrimidin-2-yl)phenyl-(E)-non-2-enoat;
4-(5-[4-(Hexyloxy)butyloxy]pyrimidin-2-yl)phenyl-(E)-dec-2-enoat;
4-(5-[4-(Hexyloxy)butyloxy]pyrimidin-2-yl)phenyl-(E)-undec-2-enoat;
4-(5-[4-(Hexyloxy)butyloxy]pyrimidin-2-yl)phenyl-(E)-dodec-2-enoat;
4-(5-[4-(Propyloxy)butyloxy]pyrimidin-2-yl)phenyl-(Z)-oct-3-enoat;
4-(5-[4-(Propyloxy)butyloxy]pyrimidin-2-yl)phenyl-(E)-oct-4-enoat;
4-(5-[4-(Propyloxy)butyloxy]pyrimidin-2-yl)phenyl-(Z)-oct-5-enoat;
4-(5-[4-(Propyloxy)butyloxy]pyrimidin-2-yl)phenyl-(E)-oct-6-enoat;
4-(5-[4-(Propyloxy)butyloxy]pyrimidin-2-yl)phenyl-oct-7-enoat.

### Beispiel 5

Zu einer Lösung von 1,0 g 4-(5-[4-(Propyloxy)butyloxy]pyrimidin-2-yl)-phenol, 0,05 g (E)-Oct-2-en-1-ol und 0,05 g Triphenylphosphin in 25 ml Tetrahydrofuran wurde unter Rühren innert 5 Minuten 0,08 g Azodicarbonsäurediethylester gegeben. Das Reaktionsgemisch wurde über Nacht weiter gerührt, mit 1 g Kieselgel versetzt und dann eingeengt. Chromatographie des Rückstandes an Kieselgel mit Hexan/Ethylacetat (Vol. 9:1) und Umkristallisation der gemäss Dünnschichtchromatographie reinen Fraktionen aus Ethylalkohol ergab 0,05 g 2-(4-[(E)-Oct-2-enyloxy]phenyl)-5-(4-[propyloxy]butyloxy)pyrimidin; Smp. (C-S_{C}) 75°C, Phasenübergang (S_{C}-N) 77°C, Klp. (N-I) 79°C.

In analoger Weise können folgende Verbindungen hergestellt werden:
2-(4-[(E)-Hex-2-enyloxy]phenyl)-5-(4-[propyloxy]butyloxy)pyrimidin;
2-(4-[(E)-Hept-2-enyloxy]phenyl)-5-(4-[propyloxy]butyloxy)pyrimidin;
2-(4-[(E)-Non-2-enyloxy]phenyl)-5-(4-[propyloxy]butyloxy)pyrimidin;
2-(4-[(E)-Dec-2-enyloxy]phenyl)-5-(4-[propyloxy]butyloxy)pyrimidin;
2-(4-[(E)-Undec-2-enyloxy]phenyl)-5-(4-[propyloxy]butyloxy)pyrimidin;
2-(4-[(E)-Dodec-2-enyloxy]phenyl)-5-(4-[propyloxy]butyloxy)pyrimidin;
2-(4-[(E)-Hex-2-enyloxy]phenyl)-5-(4-[butyloxy]butyloxy)pyrimidin;
2-(4-[(E)-Hept-2-enyloxy]phenyl)-5-(4-[butyloxy]butyloxy)pyrimidin;
2-(4-[(E)-Oct-2-enyloxy]phenyl)-5-(4-[butyloxy]butyloxy)pyrimidin;
2-(4-[(E)-Non-2-enyloxy]phenyl)-5-(4-[butyloxy]butyloxy)pyrimidin;
2-(4-[(E)-Dec-2-enyloxy]phenyl)-5-(4-[butyloxy]butyloxy)pyrimidin;
2-(4-[(E)-Undec-2-enyloxy]phenyl)-5-(4-[butyloxy]butyloxy)pyrimidin;
2-(4-[(E)-Dodec-2-enyloxy]phenyl)-5-(4-[butyloxy]butyloxy)pyrimidin;
2-(4-[(E)-Hex-2-enyloxy]phenyl)-5-(4-[pentyloxy]butyloxy)pyrimidin;
2-(4-[(E)-Hept-2-enyloxy]phenyl)-5-(4-[pentyloxy]butyloxy)pyrimidin;
2-(4-[(E)-Oct-2-enyloxy]phenyl)-5-(4-[pentyloxy]butyloxy)pyrimidin;
2-(4-[(E)-Non-2-enyloxy]phenyl)-5-(4-[pentyloxy]butyloxy)pyrimidin;
2-(4-[(E)-Dec-2-enyloxy]phenyl)-5-(4-[pentyloxy]butyloxy)pyrimidin;
2-(4-[(E)-Undec-2-enyloxy]phenyl)-5-(4-[pentyloxy]butyloxy)pyrimidin;
2-(4-[(E)-Dodec-2-enyloxy]phenyl)-5-(4-[pentyloxy]butyloxy)pyrimidin;
2-(4-[(E)-Hex-2-enyloxy]phenyl)-5-(4-[hexyloxy]butyloxy)pyrimidin;
2-(4-[(E)-Hept-2-enyloxy]phenyl)-5-(4-[hexyloxy]butyloxy)pyrimidin;
2-(4-[(E)-Oct-2-enyloxy]phenyl)-5-(4-[hexyloxy]butyloxy)pyrimidin;
2-(4-[(E)-Non-2-enyloxy]phenyl)-5-(4-[hexyloxy]butyloxy)pyrimidin;
2-(4-[(E)-Dec-2-enyloxy]phenyl)-5-(4-[hexyloxy]butyloxy)pyrimidin;
2-(4-[(E)-Undec-2-enyloxy]phenyl)-5-(4-[hexyloxy]butyloxy)pyrimidin;
2-(4-[(E)-Dodec-2-enyloxy]phenyl)-5-(4-[hexyloxy]butyloxy)pyrimidin;
2-(4-[(Z)-Oct-3-enyloxy]phenyl)-5-(4-[propyloxy]butyloxy)pyrimidin, Smp. (C-I) 71°C, Klp. (Sc-I) 59°C;
2-(4-[(E)-Oct-4-enyloxy]phenyl)-5-(4-[propyloxy]butyloxy)pyrimidin, Smp. (C-I) 88°C, S_{C}-N, 72°C, Klp. (N-I) 75°C;
2-(4-[(Z)-Oct-5-enyloxy]phenyl)-5-(4-[propyloxy]butoxy)pyrimidin, Smp. (C-I) 76°C, S_{C}-N, 64°C, Klp. (N-I) 65°C;
2-(4-[(E)-Oct-6-enyloxy]phenyl)-5-(4-[propyloxy]butoxy)pyrimidin, Smp. (C-I) 70°C, S_{C}-N, 64°C, Klp. (N-I) 77°C;;
2-(4-[7-Octenyloxy]phenyl)-5-(4-[propyloxy]butyloxy)pyrimidin.

### Beispiel 6

Zu einer Lösung von 1,0 g 5-Hydroxy-2-(4-[4-(propyloxy)butyloxy]phenyl)-pyrimidin, 0,05 g Octansäure und 0,05 g 4-(Dimethylamino)pyridin in 25 ml Dichlormethan wurde unter Rühren innert 5 Minuten 0,08 g N,N'-Dicyclohexylcarbodiimid gegeben. Das Reaktionsgemisch wurde über Nacht weiter gerührt, filtriert und das Filtrat eingeengt. Chromatographie des Rückstandes an Kieselgel mit Hexan/Ethylacetat (Vol. 9:1) und Umkristallisation der gemäss Dünnschichtchromatographie reinen Fraktionen ergab 0,05 g 2-(4-[4-(Propyloxy)butyloxy]phenyl)pyrimidin-5-yl-octanoat; Smp. (C-S_{C}) 66°C, Phasenübergang (S_{C}-N) 67°C, Klp. (N-I) 76°C.

Das als Ausgangsmaterial verwendete 5-Hydroxy-2-(4-[4-(propyloxy)-butyloxy]phenyl)pyrimidin wird wie folgt hergestellt:
a) Eine Lösung von 1,1 g 4-(5-Benzyloxypyrimidin-2-yl)phenol und 2,2 g 4-Chlor-1-butanol in 25 ml Ethylmethylketon wurde mit 2,2 g fein pulverisiertem Kaliumcarbonat versetzt und das Gemisch über Nacht unter leichtem Rückfluss erwärmt. Die Suspension wurde filtriert, und das Filtrat im Vakuum eingeengt. Chromatographie des Rückstands an Kieselgel mit Hexan/Ethylacetat (Vol. 1:1) ergab 0,5 g 5-Benzyloxy-2-(4-[4-hydroxybutyloxy]phenyl)pyrimidin mit Smp. 133°C.
b) Eine Suspension von 0,5 g 5-Benzyloxy-2-(4-[4-hydroxybutyloxy]phenyl)-pyrimidin und 1,0 g 4-Toluol-sulfonsäure-propylester in 25 ml 1,2-Dimethoxyethan wurde unter Stickstoffbegasung bei 0°C innert 5 Minuten mit 0,5 g Kalium-tert.-butylat versetzt. Das Reaktionsgemisch wurde über Nacht bei Raumtemperatur gerührt, auf 50 ml Wasser gegossen und dann der Niederschlag filtriert. Umkristallisation des Rohproduktes aus Ethylalkohol ergab 0,3 g reines 5-Benzyloxy-2-(4-[4-(propyloxy)butyloxy]-phenyl)pyrimidin mit einem Smp. von 101-102°C.
c) Ein Gemisch aus 0,3 g 5-Benzyloxy-2-(4-[4-(propyloxy)butyloxy]phenyl)-pyrimidin, 2 Tropfen Essigsäure und 25 ml Ethylacetat wurde mit 0,03 g Palladium auf Aktivkohle (10%) versetzt und bei Normaldruck und Raumtemperatur bis zum Stillstand der Wasserstoffaufnahme hydriert. Das anorganische Material wurde über Sillit filtriert und das Filtrat eingeengt. Umkristallisation des Rohproduktes aus Hexan ergab 0,22 g reines 5-Hydroxy-2-(4-[4-(propyloxy)butyloxy]phenyl)pyrimidin mit Smp. 79-80°C.

In analoger Weise können folgende Verbindungen hergestellt werden:
2-(4-[4-(Propyloxy)butyloxy]phenyl)pyrimidin-5-yl-hexanoat;
2-(4-[4-(Propyloxy)butyloxy]phenyl)pyrimidin-5-yl-heptanoat;
2-(4-[4-(Propyloxy)butyloxy]phenyl)pyrimidin-5-yl-nonanoat;
2-(4-[4-(Propyloxy)butyloxy]phenyl)pyrimidin-5-yl-decanoat;
2-(4-[4-(Propyloxy)butyloxy]phenyl)pyrimidin-5-yl-undecanoat;
2-(4-[4-(Propyloxy)butyloxy]phenyl)pyrimidin-5-yl-dodecanoat;
2-(4-[2-(Pentyloxy)ethoxy]phenyl)pyrimidin-5-yl-hexanoat;
2-(4-[2-(Pentyloxy)ethoxy]phenyl)pyrimidin-5-yl-heptanoat;
2-(4-[2-(Pentyloxy)ethoxy]phenyl)pyrimidin-5-yl-octanoat;
2-(4-[2-(Pentyloxy)ethoxy]phenyl)pyrimidin-5-yl-nonanoat;
2-(4-[2-(Pentyloxy)ethoxy]phenyl)pyrimidin-5-yl-decanoat;
2-(4-[2-(Pentyloxy)ethoxy]phenyl)pyrimidin-5-yl-undecanoat;
2-(4-[2-(Pentyloxy)ethoxy]phenyl)pyrimidin-5-yl-dodecanoat;
2-(4-[6-(Methoxy)hexyloxy]phenyl)pyrimidin-5-yl-hexanoat;
2-(4-[6-(Methoxy)hexyloxy]phenyl)pyrimidin-5-yl-heptanoat;
2-(4-[6-(Methoxy)hexyloxy]phenyl)pyrimidin-5-yl-octanoat;
2-(4-[6-(Methoxy)hexyloxy]phenyl)pyrimidin-5-yl-nonanoat;
2-(4-[6-(Methoxy)hexyloxy]phenyl)pyrimidin-5-yl-decanoat;
2-(4-[6-(Methoxy)hexyloxy]phenyl)pyrimidin-5-yl-undecanoat;
2-(4-[6-(Methoxy)hexyloxy]phenyl)pyrimidin-5-yl-dodecanoat.

### Beispiel 7

Zu einer Lösung von 1,0 g 5-Hydroxy-2-(4-[4-(propyloxy)butyloxy]phenyl)-pyrimidin, 0,05 g (E)-Oct-2-en-1-ol und 0,05 g Triphenylphosphin in 25 ml Tetrahydrofuran wird unter Rühren innert 5 Minuten 0,08 g Azodicarbonsäure-diethylester gegeben. Das Reaktionsgemisch wird über Nacht weiter gerührt, mit 1 g Kieselgel versetzt, filtriert und das Filtrat eingeengt. Chromatographie des Rückstandes an Kieselgel mit Hexan/Ethylacetat (Vol. 9:1) und Umkristallisation der gemäss Dünnschichtchromatographie reinen Fraktionen aus Ethylalkohol ergibt 5-[(E)-Oct-2-enyloxy]-2-(4-[4-(propyloxy)butyloxy]phenyl)pyrimidin.

In analoger Weise können folgende Verbindungen hergestellt werden:
5-[(E)-Hex-2-enyloxy]-2-(4-[4-(propyloxy)butyloxy]phenyl)pyrimidin;
5-[(E)-Hept-2-enyloxy]-2-(4-[4-(propyloxy)butyloxy]phenyl)pyrimidin;
5-[(E)-Non-2-enyloxy]-2-(4-[4-(propyloxy)butyloxy]phenyl)pyrimidin;
5-[(E)-Dec-2-enyloxy]-2-(4-[4-(propyloxy)butyloxy]phenyl)pyrimidin;
5-[(E)-Undec-2-enyloxy]-2-(4-[4-(propyloxy)butyloxy]phenyl)pyrimidin;
5-[(E)-Dodec-2-enyloxy]-2-(4-[4-(propyloxy)butyloxy]phenyl)pyrimidin;
5-[(E)-Hex-2-enyloxy]-2-(4-[4-(butyloxy)butyloxy]phenyl)pyrimidin;
5-[(E)-Hept-2-enyloxy]-2-(4-[4-(butyloxy)butyloxy]phenyl)pyrimidin;
5-[(E)-Oct-2-enyloxy]-2-(4-[4-(butyloxy)butyloxy]phenyl)pyrimidin;
5-[(E)-Non-2-enyloxy]-2-(4-[4-(butyloxy)butyloxy]phenyl)pyrimidin;
5-[(E)-Dec-2-enyloxy]-2-(4-[4-(butyloxy)butyloxy]phenyl)pyrimidin;
5-[(E)-Undec-2-enyloxy]-2-(4-[4-(butyloxy)butyloxy]phenyl)pyrimidin;
5-[(E)-Dodec-2-enyloxy]-2-(4-[4-(butyloxy)butyloxy]phenyl)pyrimidin;
5-[(E)-Hex-2-enyloxy]-2-(4-[4-(pentyloxy)butyloxy]phenyl)pyrimidin;
5-[(E)-Hept-2-enyloxy]-2-(4-[4-(pentyloxy)butyloxy]phenyl)pyrimidin;
5-[(E)-Oct-2-enyloxy]-2-(4-[4-(pentyloxy)butyloxy]phenyl)pyrimidin;
5-[(E)-Non-2-enyloxy]-2-(4-[4-(pentyloxy)butyloxy]phenyl)pyrimidin;
5-[(E)-Dec-2-enyloxy]-2-(4-[4-(pentyloxy)butyloxy]phenyl)pyrimidin;
5-[(E)-Undec-2-enyloxy]-2-(4-[4-(pentyloxy)butyloxy]phenyl)pyrimidin;
5-[(E)-Dodec-2-enyloxy]-2-(4-[4-(pentyloxy)butyloxy]phenyl)pyrimidin;
5-[(E)-Hex-2-enyloxy]-2-(4-[4-(hexyloxy)butyloxy]phenyl)pyrimidin;
5-[(E)-Hept-2-enyloxy]-2-(4-[4-(hexyloxy)butyloxy]phenyl)pyrimidin;
5-[(E)-Oct-2-enyloxy]-2-(4-[4-(hexyloxy)butyloxy]phenyl)pyrimidin;
5-[(E)-Non-2-enyloxy]-2-(4-[4-(hexyloxy)butyloxy]phenyl)pyrimidin;
5-[(E)-Dec-2-enyloxy]-2-(4-[4-(hexyloxy)butyloxy]phenyl)pyrimidin;
5-[(E)-Undec-2-enyloxy]-2-(4-[4-(hexyloxy)butyloxy]phenyl)pyrimidin;
5-[(E)-Dodec-2-enyloxy]-2-(4-[4-(hexyloxy)butyloxy]phenyl)pyrimidin;
5-[(Z)-Hex-3-enyloxy]-2-(4-[4-(propyloxy)butyloxy]phenyl)pyrimidin;
5-[(Z)-Hept-3-enyloxy]-2-(4-[4-(propyloxy)butyloxy]phenyl)pyrimidin;
5-[(Z)-Oct-3-enyloxy]-2-(4-[4-(propyloxy)butyloxy]phenyl)pyrimidin;
5-[(Z)-Non-3-enyloxy]-2-(4-[4-(propyloxy)butyloxy]phenyl)pyrimidin;
5-[(Z)-Dec-3-enyloxy]-2-(4-[4-(propyloxy)butyloxy]phenyl)pyrimidin;
5-[(Z)-Undec-3-enyloxy]-2-(4-[4-(propyloxy)butyloxy]phenyl)pyrimidin;
5-[(Z)-Dodec-3-enyloxy]-2-(4-[4-(propyloxy)butyloxy]phenyl)pyrimidin;
5-[(E)-Oct-4-enyloxy]-2-(4-[4-(propyloxy)butyloxy]phenyl)pyrimidin;
5-[(Z)-Oct-5-enyloxy]-2-(4-[4-(propyloxy)butyloxy]phenyl)pyrimidin;
5-[(E)-Oct-6-enyloxy]-2-(4-[4-(propyloxy)butyloxy]phenyl)pyrimidin;
5-[7-Octenyloxy]-2-(4-[4-(propyloxy)butyloxy]phenyl)pyrimidin.

### Beispiel 8

Zur Untersuchung der Eigenschaften der Verbindungen der Formel I wird eine Grundmischung (GM) hergestellt und jeweils 15% einer Verbindung der Formel I zugemischt. Die Phasenübergangstemperaturen dieser Mischungen werden bestimmt, die Kristallisationstemperatur T_{c} wird dabei aus Leitfähigkeitsdaten bestimmt. Die Schaltzeiten werden bei 25°C (10 Vpp/µ, Zeit vom Start des Pulses bis zum Maximum des Stromes) gemessen. Die Messwerte sind in der Tabelle 1 zusammengefasst.

### Grundmischung (GM)

- 16 Gew.-%: 4-[trans-4-([(R)-2-Fluorhexanoyl]oxy)cyclohexyl]phenyl 2,3-difluor-4-(octyloxy)benzoat
- 24 Gew.-%: 2-[4-(Hexyloxy)phenyl]-5-nonylpyrimidin
- 24 Gew.-%: 2-[4-(Nonyloxy)phenyl]-5-nonylpyrimidin
- 12 Gew.-%: 2-[4-(Nonyloxy)phenyl]-5-octylpyrimidin
- 12 Gew.-%: 2-[4-(Heptyloxy)phenyl]-5-heptylpyrimidin
- 12 Gew.-%: 2-[4-(Decyloxy)phenyl]-5-octylpyrimidin

**Tabelle 1**

| | **C/S**_{**X**}**-S**_{**C***} °C | **S**_{**C***}**-S**_{**A**}**/N*** °C | **S**_{**A**}**-N** | **N**_{*****}**-I** °C | **P**_{**S**} nC/cm² | τ µsec | 2θ ° |
|---|---|---|---|---|---|---|---|
| Grundmischung | -7,6 | 60,6 | 67,7 | 74,6 | 19,0 | 120 | 52,5 |
| GM +15 Gew.-% (A) | -4,3 | 62,1 | - | 72,8 | 17,8 | 155 | 59,4 |
| GM +15 Gew.-% (B) | -5,9 | 57,2 | 64,5 | 69,8 | 16,2 | 128 | 49,6 |
| GM +15 Gew.-% (C) | -4,0 | 60,1 | - | 70,7 | 17,2 | 165 | 57,6 |
| GM +15 Gew.-% (D) | -9,9 | 43,6 | 55,6 | 59,9 | 12,8 | 102 | 39,7 |
| GM +15 Gew.-% (E) | -9,4 | 61,6 | 62,8 | 72,1 | 19,0 | 143 | 57,4 |
| GM +15 Gew.-% (F) | -11,0 | 61,8 | - | 73,0 | 17,3 | 152 | 58,9 |
| GM +15 Gew.-% (G) | - | 54,0 | 70,9 | 73,7 | 13,0 | 100 | 46,6 |
| GM +15 Gew.-% (H) | - | 53,7 | 66,1 | 69,2 | 14,1 | 106 | 45,3 |
| GM +15 Gew.-% (I) | - | 52,7 | 60,2 | 66,5 | 16,9 | 119 | 49,1 |
| GM +15 Gew.-% (K) | - | 56,1 | 60,2 | 67,0 | 15,7 | 132 | 53,0 |

| | | | | | | | |
|---|---|---|---|---|---|---|---|
| A: 2-[4-(Octyloxy)phenyl]-5-[4-(propyloxy)butyloxy]pyrimidin | | | | | | | |
| B: 2-(4-Nonylphenyl)-5-[4-(propyloxy)butyloxy]pyrimidin | | | | | | | |
| C: 2-{4-[4-(Propyloxy)butyloxy]phenyl}-5-[4-(propyloxy)butyloxy]pyrimidin | | | | | | | |
| D: 2-{4-[5-(Propyloxy)pentyl]phenyl}-5-[4-(propyloxy)butyloxy]pyrimidin | | | | | | | |
| E: 2-[4-(Octanoyloxy)phenyl]-5-[4-(propyloxy)butyloxy]pyrimidin | | | | | | | |
| F: 2-{4-[4-(Propyloxy)butyloxy]phenyl}-5-(octanoyloxy)pyrimidin | | | | | | | |
| G: 2-(4-Nonylphenyl)-5-[6-(methoxy)hexyloxy]pyrimidin | | | | | | | |
| H: 2-(4-Nonylphenyl)-5-[5-(ethoxy)pentyloxy]pyrimidin | | | | | | | |
| I: 2-(4-Nonylphenyl)-5-[3-(butyloxy)propyloxy]pyrimidin | | | | | | | |
| K: 2-(4-Nonylphenyl)-5-[2-(pentyloxy)ethoxy]pyrimidin | | | | | | | |

## Patentansprüche

1. Verbindungen der allgemeinen Formel 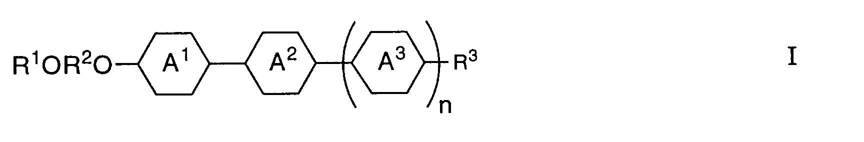 worin
R¹ geradkettiges, unsubstituiertes oder ein- oder mehrfach mit Halogen substituiertes Alkyl oder Alkenyl mit 1 bis 8 Kohlenstoffatomen;
R² geradkettiges, unsubstituiertes oder ein- oder mehrfach mit Halogen substituiertes Alkylen mit 2 bis 8 Kohlenstoffatomen;
R³ geradkettiges, unsubstituiertes oder ein- oder mehrfach mit Halogen substituiertes Alkyl, Alkoxy, Alkanoyloxy, Alkenyl, Alkenyloxy oder Alkenoyloxy mit 4 bis 12 Kohlenstoffatomen, in welchen eine oder mehrere nicht benachbarte Methylengruppen durch -O- ersetzt sein können; und
A¹, A² und A³ unabhängig voneinander unsubstituiertes oder ein- oder mehrfach mit Halogen substituiertes 1,4-Phenylen, oder Pyridin-2,5-diyl oder Pyrimidin-2,5-diyl, und A³ auch trans-1,4-Cyclohexylen bedeuten;
und
n 0 oder 1 ist;
mit der Massgabe, dass mindestens einer der Ringe A¹, A² oder A³ Pyridin-2,5-diyl oder Pyrimidin-2,5-diyl bedeutet; und falls n = 0 und A² Pyrimidin-2,5-diyl ist, R³ Alkenyl, Alkenyloxy, Alkanoyloxy oder Alkenoyloxy darstellt.

2. Verbindungen gemäss Anspruch 1 der allgemeinen Formeln: 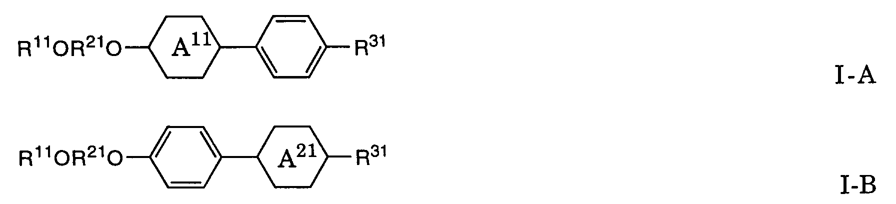 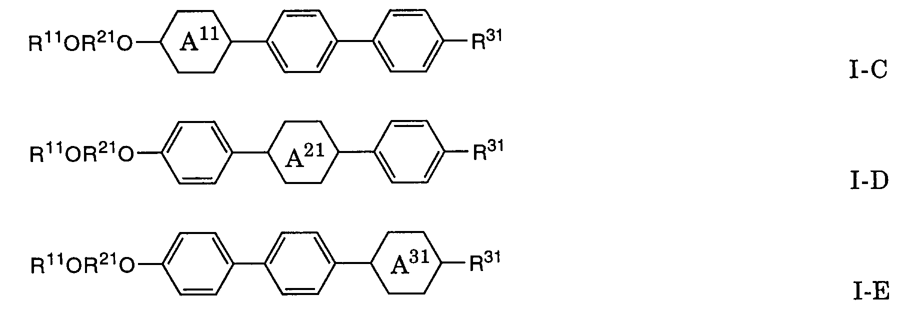 worin
R¹¹ geradkettiges, unsubstituiertes Alkyl oder Alkenyl mit 3 bis 6 Kohlenstoffatomen;
R²¹ geradkettiges, unsubstituiertes Alkylen mit 2 bis 4 Kohlenstoffatomen;
A¹¹, A²¹, A³¹ Pyridin-2,5-diyl oder Pyrimidin-2,5-diyl; und
R³¹ geradkettiges, unsubstituiertes Alkyl, Alkenyl, Alkoxy, Alkenyloxy, Alkanoyloxy, Alkenoyoxy mit 6 bis 10 Kohlenstoffatomen bedeuten.

3. Verbindungen gemäss Anspruch 2, dadurch gekennzeichnet, dass der Pyridin- bzw. der Pyrimidinring jeweils in 5-Stellung mit dem Rest R¹¹OR²¹O- bzw. dem Rest R³¹ verknüpft ist.

4. Verbindungen gemäss einem der Ansprüche 2 oder 3 der allgemeinen Formel 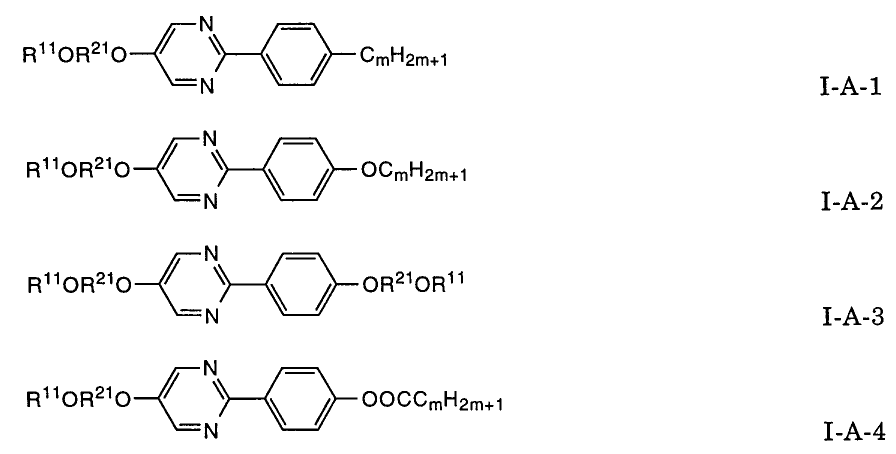 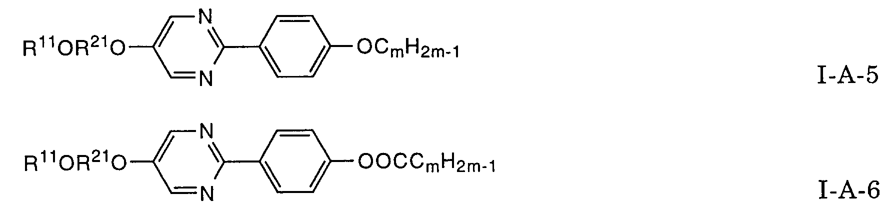 worin m eine ganze Zahl von 4 bis 8 bedeutet und den Resten R¹¹ und R²¹ die in Anspruch 2 genannten Bedeutungen zukommen.

5. Verbindungen gemäss einem der Ansprüche 2 oder 3 der allgemeinen Formel 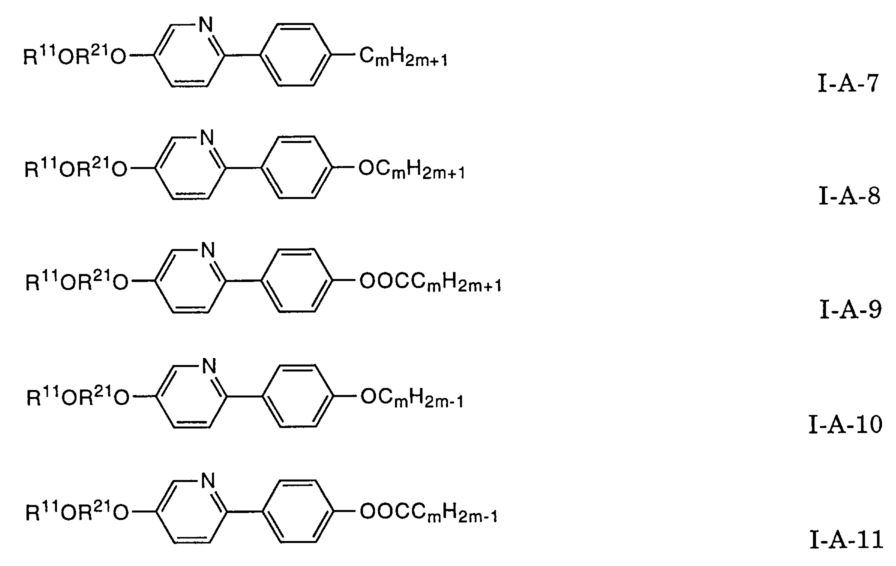 worin m eine ganze Zahl von 4 bis 8 bedeutet und den Resten R¹¹ und R²¹ die in Anspruch 2 genannten Bedeutungen zukommen.

6. Verbindungen gemäss einem der Ansprüche 2 oder 3 der allgemeinen Formel 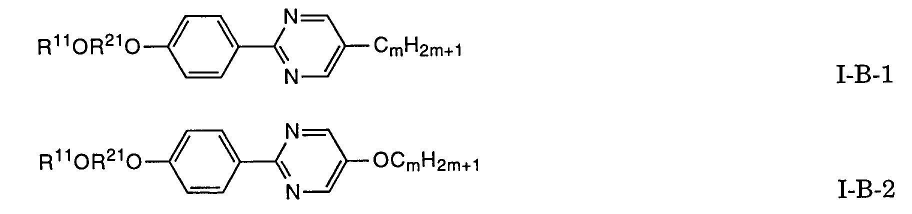 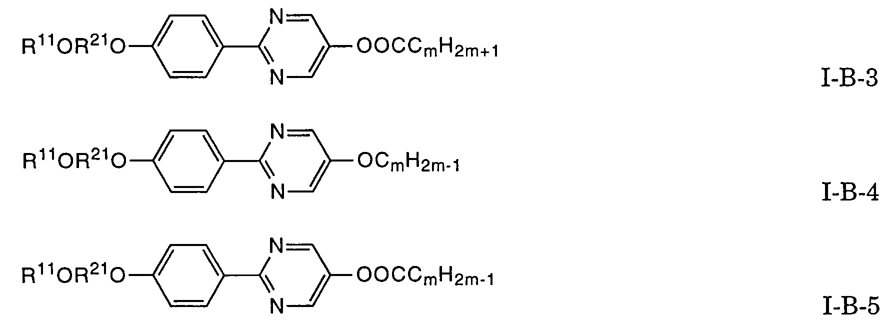 worin m eine ganze Zahl von 4 bis 8 bedeutet und den Resten R¹¹ und R²¹ die in Anspruch 2 genannten Bedeutungen zukommen.

7. Verbindungen gemäss einem der Ansprüche 2 oder 3 der allgemeinen Formel 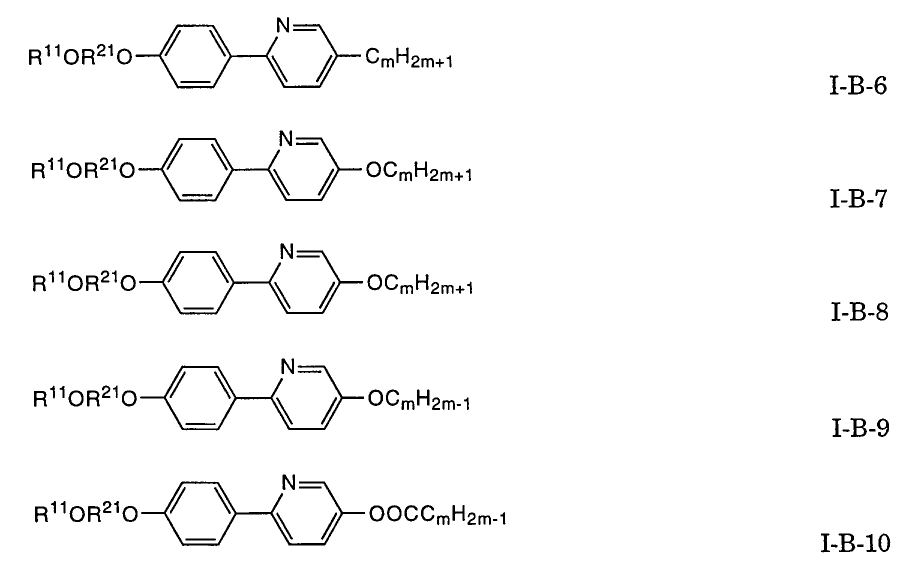 worin m eine ganze Zahl von 4 bis 8 bedeutet und den Resten R¹¹ und R²¹ die in Anspruch 2 genannten Bedeutungen zukommen.

8. Verbindungen gemäss einem der Ansprüche 1 bis 7, dadurch gekennzeichnet, dass die Reste R² bzw. R²¹ 1,4-Butylen bedeuten.

9. Flüssigkristalline Mischungen enthaltend mindestens zwei Komponenten, wovon mindestens eine Komponente eine Verbindung der in Anspruch 1 definierten allgemeinen Formel I ist.

10. Verwendung der Verbindungen der in Anspruch 1 definierten Formel I für elektro-optische Vorrichtungen.
